# EUROPÄISCHE PATENTSCHRIFT

(11) **EP 2 209 478 B1**
(45) Veröffentlichungstag und Bekanntmachung des Hinweises auf die Patenterteilung: **22.05.2013**
(21) Anmeldenummer: 08850648.0
(22) Anmeldetag: 11.11.2008
(51) Int. Cl.: A61K 31/765, A61K 31/795, C08G 14/08, C08G 14/02, C14C 3/18, C08G 12/12, C08G 12/32, C08G 14/06, C14C 3/20

(54) **KONDENSATIONSPRODUKTE AUF DER BASIS VON BICYCLISCHEN ODER POLYCYCLISCHEN AROMATEN ODER HETEROAROMATEN**
CONDENSATION PRODUCTS BASED ON BICYCLIC OR POLYCYCLIC AROMATIC OR HETEROAROMATIC COMPOUNDS
PRODUITS DE CONDENSATION À BASE D'AROMATES OU D'HÉTÉROAROMATES BICYCLIQUES OU POLYCYCLIQUES

(30) Priorität: 14.11.2007 EP 07120669
(43) Veröffentlichungstag der Anmeldung: 28.07.2010
(73) Patentinhaber: BASF SE, 67056 Ludwigshafen (DE)
(72) Erfinder: GARNIER, Sebastien, 69469 Weinheim (DE); HÜFFER, Stephan, 67063 Ludwigshafen (DE); SCHERR, Günter, 67065 Ludwigshafen (DE); ROSER, Joachim, 68161 Mannheim (DE); MROWIETZ, Ulrich, 24119 Kronshagen (DE); DOERR, Hans, Wilhelm, 63303 Dreieich (DE); CINATL, Jindrich, 63069 Offenbach (DE); MICHAELIS, Martin, 60439 Frankfurt Am Main (DE)
(86) Internationale Anmeldenummer: PCT/EP2008/065319
(87) Internationale Veröffentlichungsnummer: WO 2009/062932

(56) Entgegenhaltungen:
- WO-A-00/09570
- WO-A-97/02216
- WO-A-2007/131813
- CH-A- 139 645
- DE-A1- 3 341 122
- US-A- 4 362 712
- DATABASE WPI Week 200415 Thomson Scientific, London, GB; AN 2004-147033 XP002514683 -& JP 2003 160626 A (SHOWA HIGH POLYMER CO LTD) 3. Juni 2003 (2003-06-03)
- DATABASE WPI Week 197629 Thomson Scientific, London, GB; AN 1976-55705X XP002514684 & SU 487 917 A (FEDOSYUK M I) 8. Januar 1976 (1976-01-08)

## Beschreibung

Die Erfindung betrifft ein Kondensationsprodukt (synthetischer Gerbstoff), wie nachfolgend definiert, der gegebenenfalls als Gemisch mit mindestens einem weiteren Gerbstoff vorliegen kann, sowie Verfahren zur Herstellung eines solchen Kondensationsproduktes, dessen Verwendung als Arzneimittel sowie pharmazeutische Zusammensetzungen enthaltend ein solches Kondensationsprodukt. Ein weiterer Gegenstand der vorliegenden Erfindung ist die Verwendung des Kondensationsproduktes als Desinfektionsmittel beispielsweise in Tierstallungen und ein Desinfektionsmittel enthaltend ein solches Kondensationsprodukt. Ein weiterer Gegenstand der vorliegenden Erfindung ist die Verwendung des Kondensationsproduktes als Gerbstoff.

Gerbstoffe lassen sich prinzipiell in drei Hauptklassen einteilen (siehe Römpps Chemie Lexikon, 9. Auflage (1995), Georg Thieme Verlag Stuttgart, Stichwort "Gerbstoffe", Seiten 1541 bis 1542):
1. anorganische Gerbstoffe wie Chrom(III)-Salze oder Polyphosphate; 2. synthetische organische Gerbstoffe, die meist erhältlich sind durch Sulfonierung löslich gemachter Aldehyd-Kondensationsprodukte von aromatischen Grundkörpern, insbesondere von Phenol, Kresol, Naphthalin und Naphtol; sowie 3. Gerbstoffe von pflanzlichem Ursprung, wie sie in Blättern (Tee), Samen (Kaffee), Beeren, Gallen oder Hölzern vorkommen können. Im engeren Sinne versteht man unter Gerbstoffe von pflanzlichem Ursprung die sogenannten Gerbsäuren beziehungsweise Tannine.

Sowohl die Gerbstoffe pflanzlichen Ursprungs (nachfolgend als pflanzlicher oder natürlicher Gerbstoff bezeichnet) als auch die synthetischen organischen Gerbstoffe (nachfolgend als synthetischer Gerbstoff bezeichnet) werden in der Literatur teilweise mit antiviraler Wirkung in Zusammenhang gebracht. Dies trifft insbesondere auf pflanzliche oder synthetische Gerbstoffe zu, die als sogenannte Polyphenole bezeichnet werden.

Beispielsweise werden für pflanzliche Gerbstoffe wie Tannine in T. Okuda, Phytochemistry, Band 66 (2005), Seiten 2012 bis 2031 oder Fukuji et al., Antiviral Res. 11 (1989), Seiten 285 bis 298 eine antivirale Aktivität (insbesondere gegen Herpes simplex) sowie eine Antitumoraktivität dieser natürlichen Gerbstoffe beschrieben.

Weiterhin wird auch Propolis, das von Bienen aus den Knospen, Rinden und Harzen bestimmter Bäume gesammelt wird und pflanzliche Gerbstoffe enthält, unter anderem eine antivirale Aktivität, beispielsweise gegen Herpes simplex, zugeschrieben. Propolis, das ein komplexes Gemisch ist und u.a. Polyphenol enthält, kann sich je nach Bienenvolk aus bis zu 200 verschiedenen Inhaltsstoffen zusammensetzen, insbesondere sind dies Chalkone, Flavanone, Flavone und Flavanole (S. Bogdanov, Schweizerisches Zentrum für Bienenforschung; Artikel erhältlich aus dem Internet; http://www.apis.admin.ch/de/bienenprodukte/docs/produkte/propolis_d.pdf).

Auch im Fall von synthetischen Gerbstoffen sind pharmazeutische Anwendungen bereits bekannt. So betrifft WO 95/14479 ein Kondensationspolymer aus aromatischen Sulfonsäuren und einem Aldehyd zur Inhibierung des HIV-Virus'. Dort wird beschrieben, dass je höher das Molekulargewicht des Polymers ist, umso größer ist dessen therapeutische Aktivität. Besonders bevorzugt werden durch molekülgrößenabhängige Trennverfahren Kondensationspolymere mit einem M_{w}-Gewicht zwischen 4000 und 12.000 g/mol erhalten. WO 95/14479 offenbart jedoch keine synthetischen Gerbstoffe, die auf Aromaten oder Heteroaromaten mit mindestens einer Carboxygruppe als Substituenten beruhen. Weiterhin enthalten diese Gerbstoffe kein Harnstoffderivat. Sinngemäßes gilt für US-A 4,604,404, worin die Verwendung von beispielsweise sulfonierten Naphthalin-Formaldehyd-Kondensationspolymeren zur Bekämpfung des Herpes simplex-Virus' beschrieben wird. Ähnliche Verbindungen bzw. deren Verwendung als Arzneimittel sind in US-A 5,088,400 und US-A 4,364,927 beschrieben.

Weiterhin beschreibt DE-A 33 41 122 äußerlich anzuwendende virucide Arzneimittel, insbesondere gegen Herpes labilis sowie Viruserkrankungen der Haut. Bei diesen Arzneimitteln handelt es sich um synthetische Gerbstoffe, hergestellt durch Kondensation von beispielsweise Harnstoff mit Phenol/Kresol, Formaldehyd sowie einem Sulfonierungsmittel.

In DE-A 10 2004 034613 werden Kondensationsprodukte beschrieben, die erhältlich sind durch Umsetzung von mindestens einem Aromaten, mindestens einem Sulfonierungsmittel, mindestens einer Carbonylverbindung und gegebenenfalls mindestens einem Harnstoffderivat. Im Anschluss an die Synthese werden die Kondensationsprodukte mindestens einem molekülgrößenabhängigen Trennverfahren unterzogen. Dabei wurde das Kondensationsprodukt in drei Fraktionen, eine hochmolekulare, eine mittelmolekulare sowie eine niedermolekulare Fraktion aufgetrennt. Es wurde festgestellt, dass die hochmolekularen Fraktionen eine verbesserte Wirksamkeit hinsichtlich der Inhibierung der Aktivität des Enzyms humane Leukozyten-Elastase aufweisen im Vergleich zu den entsprechenden mittelmolekularen Fraktionen dieser Kondensationsprodukte. DE-A 2004 034613 offenbart jedoch nicht, dass bicyclische Aromaten wie Naphthalin zwingend mit einer Carboxygruppe substituiert sein müssen.

In der internationalen Anmeldung PCT/EP2007/051884 werden zu DE-A 10 2004 034613 sinngemäße Kondensationsprodukte offenbart, die hergestellt werden durch Umsetzung von mindestens einem Aromaten oder Heteroaromaten, mindestens einer Carbonylverbindung, gegebenenfalls mindestens einem Sulfonierungsmittel und gegebenenfalls mindestens einem Harnstoffderivat. Diese Kondensationsprodukte weisen eine Mw-Wert ≥ 9000 g/mol auf (hochmolekulare Fraktion) und liegen als Gemisch mit mindestens einem weiteren Gerbstoff mit einem Mw-Wert ≤ 3000 g/mol (niedermolekulare Fraktion) vor. Bei der niedermolekularen Fraktion kann es sich um einen beliebigen Gerbstoff handeln, beispielsweise um ein Kondensationsprodukt erhältlich durch Umsetzung von Melamin und/oder Harnstoff, Glyoxal, Glyoxylsäure oder einem Alkalisalz davon sowie gegebenenfalls weiterer Komponenten wie einer aromatischen Verbindung. Als niedermolekulare Fraktion kann in diesen Gemischen auch ein Kondensationsprodukt eingesetzt werden, dass hinsichtlich der Edukte mit der hochmolekularen Fraktion übereinstimmt, jedoch einen M_{w}-Wert ≤ 3000 g/mol aufweist. In einer weiteren internationalen Anmeldung WO 2007/131813 (PCT/EP 2007/051887) werden Kondensationsprodukte offenbart, die erhältlich sind durch Umsetzung von mindestens einem Aromaten oder Heteroaromaten, die mit mindestens einer Carboxygruppe und mindestens einer Hydroxygruppe substituiert sind, mindestens einer Carbonylverbindung, gegebenenfalls mindestens einem Sulfonierungsmittel, gegebenenfalls mindestens einem Harnstoffderivat und gegebenenfalls mindestens einem weiteren Aromaten oder Heteroaromaten. In keiner der beiden vorgenannten internationalen Anmeldungen wird jedoch offenbart, dass bei der Herstellung des Kondensationsproduktes der eingesetzte Aromat oder Heteroaromat auf bicyclische oder polycyclische Verbindungen beschränkt ist, die mindestens eine Carboxygruppe enthalten. Die jeweiligen Kondensationsprodukte eignen sich insbesondere als antivirales Agens beispielsweise zur Behandlung von Endzündungen der Haut, insbesondere zur Behandlung von Herpes simplex.

In CH-A 306 965 werden Kondensationsprodukte auf der Basis von Gentisinsäure (Dihydroxybenzoesäure) sowie Formaldehyd und Schwefelsäure beschrieben. Diese Kondensationsprodukte eignen sich als Arzneimittel gegen rheumatoide Polyarthritis sowie gegen gewisse Infektionskrankheiten. Diese Kondensationsprodukte enthalten jedoch kein Harnstoffderivat als Eduktkomponente. Sinngemäße, aber etwas breiter gefasste Kondensationsprodukte zur Behandlung von Infektionskrankheiten sind in BE-A 506 674 beschrieben. Als Eduktkomponente können auch Gallussäure und Hydroxy-Naphthalincarbonsäure eingesetzt werden. Die in WO 91/07183 beschriebenen Kondensationsprodukte eignen sich zur Behandlung von Herz-Kreislauf-Krankheiten. Als Edukte werden Aromaten wie Salicylsäure, Gallussäure oder Naphthalin, Schwefelsäure und Formaldehyd eingesetzt. Auch die in WO-91/07183 beschriebenen Kondensationsprodukte enthalten kein Harnstoffderivat als Eduktkomponente.

Die deutsche Anmeldung mit der Nummer 10 2005 050 193.1, GB-A 362 797 oder EP-A 0 301 406 betreffen synthetische Gerbstoffe, insbesondere niedermolekulare Gerbstoffe, für die keine Verwendung als Arzneimittel beschrieben ist. EP-A 0 301 406 betrifft Kondensationsprodukte, erhältlich durch Umsetzung von Melamin mit Glyoxal oder Glyoxylsäure. Gegebenenfalls wird eine aromatische Verbindung mit einkondensiert, ausgewählt aus Phenolsulfonsäure, Sulfosalicylsäure, Salicylsäure oder 8-Hydroxychinolin. In GB-A 362 797 werden Kondensationsprodukte beschreiben, die hergestellt werden durch Umsetzung von Gallussäure oder Salicylsäure mit Kresol, Schwefelsäure, Harnstoff und Formaldehyd.

In WO 97/02216 wird die Behandlung von wässrigen Systemen unter Verwendung eines chemisch modifizierten Tannins beschrieben. Es wird eine Zusammensetzung bereitgestellt, die ein Hydroxylgruppen enthaltenes Tannin umfasst, welches durch die Reaktion mindestens einer dieser Hydroxylgruppen mit einer anderen Komponente chemisch modifiziert und dann derivatisiert wurde. Als mögliche Komponenten für die Modifikation werden ein Esterifizierungsmittel (zum Beispiel Essigsäureanhydrid), ein Etherifizierungsmittel (zum Beispiel Dichlormethan oder quaternäre organische Amine wie N-(3-chlor-2-hydroxypropyl)trimethylammoniumchlorid zur Bildung des entsprechenden Esters oder Ethers genannt. Die Derivatisierungsreaktion wird mit einem Aldehyd (zum Beispiel Formaldehyd) oder einem Aldehyd und mindestens einer Verbindung ausgewählt aus Ammoniak und organischen Aminen, die mindestens ein primäres oder sekundäres Stickstoffatom enthalten. Die modifizierten Tannine der WO 97/02216 sind für die Koagulation und/oder Entklebung von in wässrigem System suspendierten festen Partikeln wie beispielsweise in dem Abwasser von Farbsprühvorgängen suspendierten Farbpartikeln geeignet. Ebenso finden diese Tannine für die Deemulgierung in Öl-in-Wasser-Emulsionen Anwendung.

Die US 4,362,712 offenbart carboxylierte Naphthalin-Formaldehyd-KondensationsPolymere als Barriere für Zahnbelag (Plaque) in der Zahnmedizin. Die für die Verhinderung des Auftretens von Zahnbelag anwendbaren Zusammensetzungen umfassen Alkalimetallsalze von bestimmten carboxylierten Naphtalin-Formaldehyd-Polymeren in einem pharmazeutisch verträglichen Träger. Insbesondere umfasst eine Oralhygiene-Zusammensetzung ein Kondensationspolymer von Formaldehyd mit einer Naphtalinverbindung, die aus der Gruppe bestehend aus 1-Napthoesäure, 2-Napthoesäure, 1-Napthylessigsäure, 2-Napthylessigsäure, wobei das Polymer bestimmte sich wiederholende strukturelle Einheiten hat.

In keinem der vorgenannten Dokumente, die synthetische Gerbstoffe (Kondensationsprodukte) als solche auf der Basis von Aromaten oder deren Verwendung als Arzneimittel betreffen, wird jedoch beschrieben, dass die im jeweiligen Kondensationsprodukt eingesetzte Aromatenkomponente bicyclische oder polycyclische Aromaten oder Heteroaromaten sein können, die mit mindestens einer Carboxygruppe substituiert sein müssen beziehungsweise den Salzen davon.

Der Erfindung lag somit die Aufgabe zugrunde, weitere Arzneimittel bereitzustellen, die als antivirales Agens geeignet sind, vorzugsweise sollen diese neuen Arzneimittel eine verbesserte Wirkung gegenüber Viren wie beispielsweise dem Herpes simplex-Virus aufweisen. Erfindungsgemäß wird diese Aufgabe gelöst durch ein Kondensationsprodukt erhältlich durch Umsetzung von
a1) 1-Naphthalincarbonsäure oder einem Salz davon,
a2) mindestens einem Aldehyd ausgewählt aus Formaldehyd, Acetaldehyd und Propionaldehyd,
a3) konzentrierter Schwefelsäure,
a4) mindestens einem Harnstoffderivat ausgewählt aus Harnstoff, Melamin, 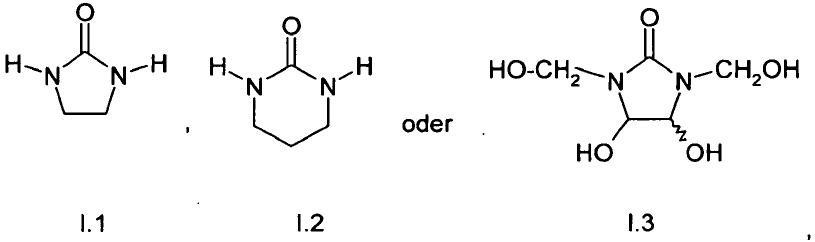 und
a5) gegebenenfalls Phenol,
oder ein physiologisch verträgliches Salz davon.

Ein Vorteil der vorliegenden Erfindung ist darin zu sehen, dass die erfindungsgemäßen Kondensationsprodukte eine deutlich verbesserte Wirksamkeit als antivirales Agens, beispielsweise gegenüber dem Herpes simplex-Virus sowie weiteren Viren, aufweisen. Die verbesserte Wirksamkeit wird insbesondere dadurch erzielt, dass ein Kondensationsprodukt hergestellt wird aus Komponente a1), wobei die Carboxygruppe auch teilweise oder vollständig in Salzform vorliegen kann, insbesondere Naphthalincarbonsäure oder Salzen der Naphthalincarbonsäure auf. Sofern bei der Herstellung der erfindungsgemäßen Kondensationsprodukte die Komponente a1) noch mit mindestens einer Komponente a5) kombiniert wird, kann die Wirksamkeit weiter erhöht werden. Die verbesserte Wirksamkeit der erfindungsgemäßen Kondensationsprodukte liegt sowohl gegenüber herkömmlichen synthetischen Gerbstoffen auf Phenolbasis oder Naphthalinbasis als auch gegenüber pflanzlichen Gerbstoffen beispielsweise auf Gallussäurebasis, wie sie in grünem Tee enthalten sind, vor.

Ein weiterer Vorteil der vorliegenden Erfindung ist darin zu sehen, dass die erfindungsgemäßen Kondensationsprodukte auch zur Inhibierung von Serinproteasen, insbesondere zur Inhibierung der Humanen Leukozyten Elastase (HLE) geeignet sind.

Sofern die erfindungsgemäßen Kondensationsprodukte in Form von Gemischen mit weiteren Gerbstoffen eingesetzt werden, so ist ein weiterer Vorteil dieser erfindungsgemäßen Gemische darin zu sehen, dass in dem Fall, in dem ein erfindungsgemäßes Kondensationsprodukt auf Formaldehyd-Basis als Gemisch mit einem weiteren synthetischen, formaldehydfreien Gerbstoff eingesetzt wird, insbesondere unter Verwendung von mindestens einem Kondensationsprodukt (C) oder (D), der Anteil an formaldehydhaltigen Komponenten in dem erfindungsgemäßen Gemisch verringert werden kann. Die Aktivität dieser Gemische ist in Abhängigkeit vom Gehalt an erfindungsgemäßem Kondensationsprodukt zumindest gleich, in der Regel jedoch besser als bei synthetischen Gerbstoffen gemäß dem Stand der Technik. Dies ist vor dem Hintergrund zu sehen, dass Formaldehyd, das ein weit verbreitetes Edukt bei der Herstellung von synthetischen Gerbstoffen ist, von der Weltgesundheitsbehörde (WHO) mittlerweile als kanzerogenverdächtig eingestuft wird. Demzufolge sollte Formaldehyd bei der Synthese möglichst vermieden werden, da bei den erhaltenen Kondensationsprodukten immer ein gewisser Formaldehyd-Restgehalt freigesetzt wird. Da aber beispielsweise auch ein Apfel in geringen Konzentrationen Formaldehyd enthält, sind dementsprechend geringe Formaldehyd-Konzentrationen in pharmazeutischen Zusammensetzungen tolerabel. Durch diese Ausführungsformen der erfindungsgemäßen Kondensationsprodukte wird jedoch der Formaldehydgehalt reduziert.

Die Komponente a1) ist 1-Naphthalincarbonsäure (α-Naphthalincarbonsäure), wobei die Carboxygruppe teilweise oder vollständig in Salzform vorliegen kann, vorzugsweise als physiologisch verträgliche Salze.

. Bevorzugte physiologisch verträgliche Salze von Komponente a1) sind Alkalimetallsalze (insbesondere das Natriumsalz oder das Kaliumsalz), und Erdalkalisalze (z.B. Magnesiumsalz). Weitere bevorzugte Salze sind Bismutsalz.

### a2) mindestens eine Carbonylverbindung

Die Carbonylverbindung ist ein Aldehyd wie Formaldehyd, Acetaldehyd oder Propionaldehyd und insbesondere Formaldehyd. Wünscht man Formaldehyd einzusetzen, so ist es bevorzugt, Formaldehyd in wässriger Lösung einzusetzen.

### a3) ein Sulfonierungsmittel

Ein geeignetes Sulfonierungsmittel ist konzentrierte Schwefelsäure, weiterhin Oleum mit einem Gehalt an SO₃ von 1 bis 30 Gew.-%, weiterhin Chlorsulfonsäure und Amidosulfonsäure. Bevorzugt sind konzentrierte Schwefelsäure und Oleum mit einem Gehalt an SO₃ von 1 bis 15 Gew.-%.

### a4) mindestens ein Harnstoffderivat

Prinzipiell sind als Komponente a4) Harnstoff und alle Derivate davon geeignet, nämlich (unsubstituierter) Harnstoff, Melamin oder die cyclischen Harnstoffderivate der Formeln I.1, I.2 oder 1.3

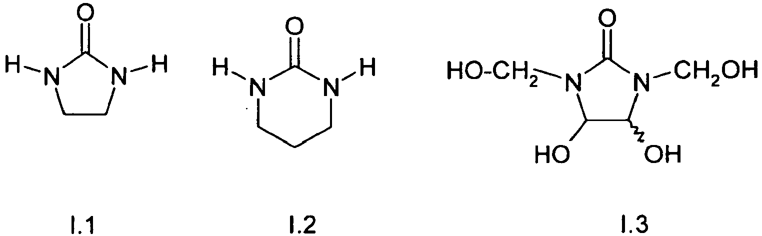

### a5) gegebenenfalls

### Phenol.

In einer bevorzugten Ausführungsform der vorliegenden Erfindung wird bei der Herstellung des Kondensationsproduktes neben den Komponenten a1), a2) und a4) auch mindestens eine Komponente a3) und mindestens eine Komponente a5) als Edukt (Reaktand) eingesetzt. In einer mehr bevorzugten Ausführungsform wird bei der Herstellung des Kondensationsproduktes jeweils mindestens eine Komponente a1) bis a5) eingesetzt. Vorzugsweise wird eine Komponente a1) und eine Komponente a5) eingesetzt.

Verfahren zur Herstellung eines erfindungsgemäßen Kondensationsproduktes sind dem Fachmann bekannt, beispielsweise werden sie in EP-A 37 250, DE-A 1 113 457. Ullmanns Encyclopedia of Industrial Chemistry, Band A15, (5.Auflage) Weinheim 1990, S.259 - 282 oder DE-A 848 823 beschrieben.

Man kann dabei die einzelnen Komponenten a1), a2), a3) und a4) sowie gegebenenfalls a5) in einer oder in mehreren Stufen umsetzen. Beispielsweise kann man zunächst
a1) mindestens einen mit -COOH substituierten bicyclischen oder polycyclischen Aromaten oder Heteroaromaten
a3) mit mindestens einem Sulfonierungsmittel umsetzen und danach im selben Gefäß ohne vorherige Isolierung mit
a2) mindestens einer Carbonylverbindung,
a4) mindestens einem Harnstoffderivat und
a5) gegebenenfalls mindestens einen weiteren Aromaten oder Heteroaromaten umsetzen.

Alternativ kann dabei die Reihenfolge der Zugabe der Komponenten a1) und a5) vertauscht sein oder a1) und a5) werden mindestens einmal in Form eines Gemisches zugegeben.

Man kann in einer anderen Ausführungsform vorgehen, indem man
a1) mindestens einen mit -COOH substituierten bicyclischen oder polycyclischen Aromaten oder Heteroaromaten
a3) mit mindestens einem Sulfonierungsmittel umsetzt, das Produkt isoliert und dann mit dem Umsetzungsprodukt von
a2) mindestens einer Carbonylverbindung,
a4) mindestens einem Harnstoffderivat und
a5) mindestens einem weiteren Aromaten oder Heteroaromaten umsetzt.

Man kann in einer Ausführungsform der vorliegenden Erfindung die Komponenten a1), a2) und a4) und gegebenenfalls a3) und a5) in jeweils einer Portion zur Umsetzung bringen.

In einer anderen Ausführungsform der vorliegenden Erfindung bringt man mindestens eine Komponente a1) bis a5) in mindestens zwei Portionen zur Umsetzung. Vorzugsweise sind dies die Komponenten a1), a2) und/oder a5), insbesondere die Komponente a2). Dies bedeutet, dass man die zweite Portion - in maximal äquimolaren- Mengen zur ersten Portion - nach Umsetzung der übrigen Komponenten in das Reaktionsgefäß zugibt und eine sogenannte Nachkondensation durchführt.

In einer speziellen Ausführungsform der vorliegenden Erfindung bringt man mehrere Komponenten a1), a2), a3) und a4) und gegebenenfalls a5) in mehreren Portionen zur Umsetzung.

In einer Ausführungsform der vorliegenden Erfindung kann man während der Umsetzung
a6) einen oder mehrere weitere Komponenten zusetzen, beispielsweise NaHSO₃, Na₂S₂O₅, KHSO₃, K₂S₂O₅, wässrige Alkalimetallhydroxidlösung, insbesondere wässrige Natronlauge und wässrige Kaliläuge, und wässriges Ammoniak. Weiterhin sind auch Komplexbildner als Komponente a6) geeignet, beispielsweise Komplexbildner auf Basis Ethylendiamin-Tetraessigsäure. Ein Beispiel für einen solchen Komplexbildner ist das Handelsprodukt Trilon B (BASF AG, Ludwigshafen, Deutschland), das Ethylendiamintetraacetat (EDTA) enthält. Diese Komplexe sind stabil gegen Temperatur- (vorzugsweise bis mindestens 100 °C) und pH-Wert-Schwankungen. Die Komplexbildner fördern die Bildung von wasserlöslichen Komplexen mit Ionen. Die Komponente a6) dient insbesondere zur Einstellung des pH-Wertes sowie der Steuerung der Löslichkeit des Endprodukts

Sofern die Komponente a6) in den erfindungsgemäßen Kondensationsprodukten enthalten ist, beträgt das Verhältnis a1) zu a6) 10 bis 99 Gew.-% zu 1 bis 90 Gew.-%, insbesondere 30 bis 80 Gew.-% zu 20 bis 70 Gew.-%.

In einer Ausführungsform der vorliegenden Erfindung wählt man die Komponenten a1) bis a6) in folgendem Verhältnis:
a1) im Bereich von insgesamt 10 bis 70 Gew.-% (Gewichtsprozent), bevorzugt insgesamt 20 bis 60 Gew.-%, besonders bevorzugt insgesamt 35 bis 50 Gew.%,
a2) im Bereich von insgesamt 5 bis 40 Gew.-%, bevorzugt insgesamt 10 bis 30 Gew.-%, besonders bevorzugt insgesamt 15 bis 25 Gew.-%,
a3) gegebenenfalls im Bereich von insgesamt 5 bis 50 Gew.-%, bevorzugt insgesamt 10 bis 40 Gew.-%, besonders bevorzugt insgesamt 20 bis 30 Gew.-%, wobei Sulfonierungsmittel stets als SO₃ berechnet werden,
a4) im Bereich von 0 bis insgesamt 30 Gew.-%, bevorzugt insgesamt 10 bis 25 und besonders bevorzugt 15 bis 25 Gew.-%,
a5) gegebenenfalls im Bereich von insgesamt 10 bis 70 Gew.%, bevorzugt insgesamt 20 bis 60 Gew.-%, besonders bevorzugt insgesamt 35 bis 50 Gew.%,
wobei Gew.-% jeweils auf die Summe aller Komponenten a1) bis a4), sowie gegebenenfalls a5), bezogen sind,
a6) im Bereich von 0 bis insgesamt 30 Gew.-%, bevorzugt bis insgesamt 25 Gew.% und besonders bevorzugt insgesamt bis 20 Gew.%,
wobei die Gew.%-Angaben von a6) auf die Summe der Komponenten a1), a2) und a4), gegebenenfalls a1) bis a5), bezogen sind.

Man kann beispielsweise bei Temperaturen im Bereich von 40 bis 200°C, bevorzugt 50 bis 110°C umsetzen. Üblicherweise passt man dabei die Temperatur der Umsetzung an a1) und a2) an. Wünscht man beispielsweise aromatische Alkohole umzusetzen, so ist es bevorzugt, bei Temperaturen im Bereich von 50 bis 110°C umzusetzen. Natürlich ist es auch möglich, während der Umsetzung ein gewisses Temperaturprofil einzustellen. So ist es beispielsweise möglich, zunächst bei 90 bis 100°C die Reaktion zu starten und nach einiger Zeit, beispielsweise nach 2 bis 10 Stunden, auf 40 bis 75°C abzukühlen und die Umsetzung über einen Zeitraum von beispielsweise 1 bis 10 Stunden zu vervollständigen.

Man setzt beispielsweise bei Atmosphärendruck um, kann aber, falls es gewünscht ist, auch bei höheren Drücken, beispielsweise 1,1 bis 10 bar.

Durch die oben beschriebene Umsetzung erhält man Reaktionslösungen, die üblicherweise große Mengen an Säuren wie insbesondere Schwefelsäure oder - im Falle des Einsatzes von Chlorsulfonsäure - HCl enthalten. Weiterhin können Reaktionslösungen große Mengen an Alkalimetallsulfat und/oder Alkalimetallchlorid enthalten.

Im Anschluss an die oben beschriebene Umsetzung kann man mit beispielsweise wässriger Alkalimetallhydroxidlösung oder wässrigem Ammoniak einen pH-Wert im Bereich von 3 bis 10, bevorzugt 3,5 bis 9 einstellen.

Durch Zugabe von Wasser zu durch die oben beschriebene Umsetzung erhältliche Reaktionslösungen kann man durch Verdünnen mit Wasser einen Wassergehalt im Bereich von 70 bis 95 Gew.-%, bevorzugt 75 bis 90 Gew.-% einstellen.

Aufgrund der in der Komponente a1) zwingend vorhandenen Carboxyl-Gruppe sowie in den einzelnen Komponenten eventuell vorhandenen weiteren sauren oder basischen Gruppen, umfasst die vorliegende Erfindung auch die entsprechend physiologisch oder toxikologisch verträgliche Salze der erfindungsgemäßen Kondensationsprodukte. Gegebenenfalls können die erfindungsgemäßen Kondensationsprodukte im Anschluss an deren Herstellung in physiologisch verträgliche Salze überführt werden.

Physiologisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein physiologisch verträgliches Anion oder Kation aufweisen. Geeignete physiologisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Theophyllinessigsäure, Methylen-bis-b-oxynaphthoe-, Benzolsulfon-, Benzoe-, Zitronen-, Ethansulfon-, Salicyl-, Fumar-, Glucon-, Glykol-, Isethion-, Milch-, Lactobion-, Malein-, Apfel-, Methansulfon-, Bernstein-, p-Toluolsulfon-, Wein- und Trifluoressigsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Die entsprechenden Salze der erfindungsgemäßen Kondensationsprodukte können durch herkömmliche Methoden, die dem Fachmann bekannt sind, erhalten werden, beispielsweise durch Umsetzung mit einer organischen oder anorganischen Säure oder Base in einem Lösungsmittel oder Dispergiermittel, oder durch Anionen- oder Kationenaustausch mit anderen Salzen.

Die vorliegende Erfindung schließt darüber hinaus alle Solvate der Kondensationsprodukte mit ein, beispielsweise Hydrate oder Addukte mit Alkohol, aktive Metaboliten sowie Derivate, die eine physiologisch annehmbare und abspaltbare Gruppe enthalten, beispielsweise Ester oder Amide.

Der hier verwendete Begriff "physiologisch funktionelles Derivat" bezeichnet jedes physiologisch verträgliche Derivat eines erfindungsgemäßen Kondensationsproduktes, z.B. einen Ester, der bei Verabreichung an einen Säuger, wie z.B. den Menschen, in der Lage ist, (direkt oder indirekt) ein erfindungsgemäßes Kondensationsprodukt oder einen aktiven Metaboliten hiervon zu bilden. Beispiele hierfür sind Acetyl-Phenoxyderivate, erhältlich durch Umsetzung von Essigsäureanhydrid und einer der vorhandenen Phenolgruppen.

Zu den physiologisch funktionellen Derivaten zählen auch Prodrugs der erfindungsgemäßen Kondensationsprodukte. Solche Prodrugs können in vivo zu einem erfindungsgemäßen Kondensationsprodukt metabolisiert werden. Diese Prodrugs können selbst wirksam sein oder nicht und sind ebenfalls Gegenstand der vorliegenden Erfindung.

Die erfindungsgemäßen Kondensationsprodukte können auch in verschiedenen polymorphen Formen vorliegen, z.B. als amorphe und kristalline polymorphe Formen. Alle polymorphen Formen der erfindungsgemäßen Kondensationsprodukte gehören in den Rahmen der Erfindung und sind ein weiterer Aspekt der Erfindung.

In einer weiteren Ausführungsform der vorliegenden Erfindung kann bei der Herstellung des erfindungsgemäßen Kondensationsproduktes nach Umsetzung der Komponenten a1), a2) und a4) sowie gegebenenfalls a3) und a5) ein molekülgrößenabhängiges Trennverfahren durchgeführt werden, vorzugsweise eine Ultrafiltration unter Erhalt einzelner Fraktionen des erfindungsgemäßen Kondensationsproduktes, beispielsweise einer niedermolekulare, einer mittelmolekulare sowie einer hochmolekulare Fraktion. Sinngemäßes gilt auch für die nachstehend beschriebenen Kondensationsprodukte (B). Die hochmolekulare Fraktion hat beispielsweise eine M_{w}-Wert ≥ 9000 g/mol (M_{w} = gewichtsmittleres Molekulargewicht), vorzugsweise einen M_{w}-Wert von 10.000 bis 100.000 g/mol. Die niedermolekulare Fraktion hat vorzugsweise einen M_{w}-Wert von 300 bis 3000 g/mol. Vorzugsweise beträgt in der hochmolekularen Fraktion das Verhältnis M_{w}/Mₙ < 10, insbesondere M_{w}/Mₙ < 5 (Mₙ = zahlenmittleres Molekulargewicht).

An molekülgrößenabhängigen Trennverfahren sind beispielsweise geeignet: Präparative Gelpermeationschromatographie und Membrantrennverfahren wie beispielsweise die Mikrofiltration, die Nanofiltration und insbesondere die Ultrafiltration. Auch Kombinationen von Mikrofiltration und Ultrafiltration sind geeignet. Mikrofiltrationen und Ultrafiltrationen und dafür erforderliche Membranen sind als solche bekannt und beschrieben beispielsweise in Ullmann's Encyclopedia of Industrial Chemistry, 6. Auflage, Bd. 21, Wiley-VCH Weinheim, S. 243 - 321. Nanofiltrationen und die dazu gehörigen Membranen sind als solche ebenfalls bekannt und beschrieben in R. Rautenbach, "Membranverfahren", Springer Verlag Berlin Heidelberg 1997.

Ultrafiltrationen sind als solche bekannt und werden im Allgemeinen als Querstromultrafiltrationen betrieben. Als Membranen sind handelsübliche Membranen geeignet, die beispielsweise aus organischen Materialien wie Polysulfonen oder Polyvinylidenfluorid oder bevorzugt aus anorganischen Materialien wie beispielsweise TiO₂, ZrO₂ oder Al₂O₃ hergestellt werden. Übliche Formen sind Kapillar-, Rohr- und Flachmembranen, letztere in Form von Membrankissen oder Spiralwickelmodulen.

Beispielsweise wendet man bei Membrantrennverfahren und insbesondere bei Ultrafiltrationen eine transmembrane Druckdifferenz, d.h. eine Druckdifferenz zwischen Feed und Permeat, im Bereich von 1 bis 200 bar, bevorzugt im Bereich von 1,2 bis 100 bar an.

In einer Ausführungsform liegt die Temperatur der nach Membrantrennverfahren behandelten Reaktionslösung im Bereich von 20 bis 70 °C, bevorzugt 25 bis 35 °C.

In einer Ausführungsform der vorliegenden Erfindung setzt man mindestens eine Membran mit einem molecular weight cut-off im Bereich von 1000 Dalton, bevorzugt 2000 Dalton, besonders bevorzugt 5000 Dalton, ganz besonders bevorzugt 7500 Dalton und noch mehr bevorzugt von 15.000 Dalton. Der molecular weight cut-off wird auch als Trenngrenze bezeichnet.

In einer Ausführungsform der vorliegenden Erfindung führt man die Ultrafiltration so durch, dass man ein bestimmtes Massenverhältnis von Permeat zu Retentat am Ende der Ultrafiltration einstellt. Die Retentatmenge bleibt üblicherweise bei der Ultrafiltration durch ständiges Nachdosieren von Wasser konstant, die Permeatmenge steigt im Laufe der Filtrationszeit an. Übliche Werte liegen im Bereich von 0,5:1 bis 10:1, bevorzugt 0,8:1 bis 5:1, besonders bevorzugt 1,0:1 bis 3:1.

Man erhält üblicherweise visuell im Wesentlichen transparente wässrige Lösungen von Kondensationsprodukten.

Es ist möglich, die Kondensationsprodukte aus den vorstehend beschriebenen Lösungen zu isolieren, beispielsweise durch Eindampfen des Wassers oder durch Sprühtrocknung.

In einer Ausführungsform der vorliegenden Erfindung haben die Kondensationsprodukte einen Salzgehalt an anorganischen Salzen wie beispielsweise Alkalimetallsulfat und Alkalimetallchlorid von 10 ppm bis weniger als 5 Gew.-%, bevorzugt weniger als 2 Gew.-%, besonders bevorzugt weniger als 1 Gew.-% und ganz besonders bevorzugt weniger als 0,5 Gew.-%, bezogen auf das Trockengewicht von Kondensationsprodukt. Der Salzgehalt lässt sich beispielsweise durch Ionenchromatographie (IC) ermitteln, wie beispielsweise in Römpps Lexikon Chemie, 10. Auflage, Georg Thieme Verlag Stuttgart New York, Band 2, Stichwort: Ionenchromatographie beschrieben.

In einer Ausführungsform der vorliegenden Erfindung haben die Kondensationsprodukte einen Restmonomergehalt von 10 ppm bis weniger als 5 Gew.-%, bevorzugt weniger als 2 Gew.-%, bezogen auf das Trockengewicht des Kondensationsprodukts. Als Restmonomer werden im Rahmen der vorliegenden Erfindung nicht abreagierte Reaktanden a1), a2), a4) und gegebenenfalls a5) bezeichnet, die sich in Kondensationsprodukten befinden können. Der Restmonomergehalt lässt sich beispielsweise durch Gelpermeationschromatographie (GPC) oder vorzugsweise durch lonenchromatographie (IC) oder Hochdruckflüssigkeitschromatographie (HPLC) bestimmen.

In einer Ausführungsform der vorliegenden Erfindung haben erfindungsgemäße Kondensationsprodukte einen Gehalt von freier Carbonylverbindung a2) einschließlich als Hydrat vorliegender Carbonylverbindung a2) im Bereich von 1 ppm bis weniger als 0,5 Gew.-%, bevorzugt 0,1 Gew.-% oder weniger, bezogen auf das Trockengewicht von erfindungsgemäßem Kondensationsprodukt. In dieser Ausführungsform bezieht sich die Menge an freier Carbonylverbindung a2) natürlich auf die Carbonylverbindung a2), das man bei der Umsetzung von a1), a2) und a4) und gegebenenfalls a3) und a5) eingesetzt hat. Hat man mehrere Carbonylverbindungen a2) eingesetzt, so bezieht sich der Gehalt an freier Carbonylverbindung a2) auf die Summe aller Carbonylverbindungen a2), die man bei der Umsetzung von a1), a2), a3) und a4) und gegebenenfalls a5) eingesetzt hat. Die Bestimmung des Gehalts an freier Carbonylverbindung a2) kann man nach an sich bekannten Methoden durchführen. Ist Carbonylverbindung a2) bei Zimmertemperatur fest oder flüssig, so kann man den Gehalt an freier Carbonylverbindung a2) beispielsweise durch Gaschromatographie oder HPLC bestimmen. Handelt es sich bei Carbonylverbindung a2) um Formaldehyd, so lässt sich er sich beispielsweise photometrisch bestimmen. Eine besonders bevorzugte Methode zur Bestimmung von freiem Formaldehyd ist die Umsetzung mit Acetylaceton und Ammoniumacetat zu Diacetyldihydrolutidin und photometrische Messung von Diacetyldihydrolutidin bei einer Wellenlänge von 412 nm.

In einer Ausführungsform der vorliegenden Erfindung wird das vorstehend beschriebene Kondensationsprodukt als Gemisch mit mindestens einem (weiteren) Gerbstoff eingesetzt, insbesondere einem synthetischen oder pflanzlichen Gerbstoff. In einer Ausführungsform beträgt der M_{w}-Wert des weiteren Gerbstoffes ≤ 3000 g/mol, bevorzugt ist ein M_{w}-Wert zwischen 300 und 3000 g/mol.

Der weitere Gerbstoff kann sowohl ein anorganischer Gerbstoff, ein pflanzlicher Gerbstoff oder ein synthetischer Gerbstoff sein (siehe hierzu die vorstehend aufgeführte Definition gemäß Römpps Chemie Lexikon, 9. Auflage (1995), Georg Thieme Verlag, Stuttgart, Stichwort: "Gerbstoffe", Seiten 1541 bis 1542). Vorzugsweise werden als weitere Gerbstoffe pflanzliche oder synthetische Gerbstoffe verwendet, besonders bevorzugt sind hierbei synthetische Gerbstoffe. In einer Ausführungsform sind solche Gerbstoffe bevorzugt, die kein Formaldehyd als Edukt enthalten.

Der weitere Gerbstoff kann in beliebiger Konzentration dem erfindungsgemäßen Kondensationsprodukt zugegeben werden. Vorzugsweise liegt das erfindungsgemäße Kondensationsprodukt in einem solchen Gemisch zu mindestens 50 Gew.-% bezogen auf die Summe der Gerbstoffe vor.

Beispiele für pflanzliche Gerbstoffe sind Tannine wie Catechine oder Gallussäurederivate wie Gallate. Pflanzliche Gerbstoffe, die auf Gallussäurederivaten (wie Gallaten) beruhen, unterscheiden sich von den erfindungsgemäßen Kondensationsprodukten insbesondere darin, dass die Letztgenannten in ihren chemischen Strukturen (eine Vielzahl von) -CR¹R²-Brücken (Vernetzungen) aufweisen, die von der eingesetzten Carbonylverbindung a2) stammen und die in pflanzlichen Gerbstoffen nicht vorhanden sind. Sofern beispielsweise Formaldehyd als Komponente a2) eingesetzt wird, weisen die Kondensationsprodukte -CH₂-Brücken auf. Pflanzliche Gerbstoffe (Gallate) sind typischerweise oligomere Systeme, während es sich bei den Kondensationsprodukten gemäß der vorliegenden Erfindung vorzugsweise um Polymere handelt. In einer Ausführungsform haben die Kondensationsprodukte ein Molekulargewicht M_{w} ≥ 800 g/mol, vorzugsweise ≥ 2500 g/mol, insbesondere 10000 bis 50000 g/mol.

Bevorzugte pflanzliche Gerbstoffe sind Tannine aus der Gruppe der Catechine, Epicatechine und Epigallocatechine und deren Gallate.

Als Tannin versteht man prinzipiell natürlich vorkommende Polyphenole, wie sie beispielsweise in T. Okuda Phytochemistry, Band 66 (2005), Seiten 2012 bis 2031 oder Römpp's Chemielexikon, 9. Auflage (1995), Georg Thieme Verlag, Stuttgart, Stichwort "Tannine", Seiten 4452 bis 4453 aufgeführt sind. Bevorzugte Tannine sind Ellagitannine und Dehydroellagitannine, insbesondere Geraniin, Dehydrogeraniin, Furosinin, Ascorgeraniin, Geraniinsäure, Mallotusinsäure, Pentagalloylglukose, Camelliatannin A, Casuariin, Euphorbin E, Camelliatannin F, Agrimoniin, Trapanin B, Oenothein A, Oenothein B oder Gemin D , Lignin und Ligninsulfonate. Weiterhin bevorzugt sind Catechine, Epicatechine und Epigallocatechine.

Beispiele für ein geeignetes Catechin oder Derivate davon umfassen insbesondere Flavan-3-ole, Flavan-3,4-diole (Leukoanthocyanidine) sowie Flavanone, Flavone, Chalkone oder Dihydrocychalkone, Epicatechine und Epigallocatechine.

Beispiele für geeignete pflanzliche Gallussäurederivate sind beispielsweise in H. Sakagami et al, Anticancer Research 17 (1997), Seiten 377 bis 380 aufgeführt. Vorzugsweise sind dies Methyl-tri-O-methylgallat, tri-O-Methylgallussäure, Methyl-tri-O-acetylgallat, Methylgallat, Ethylgallat, n-Propylgallat, Isoamylgallat, Laurylgallat, Stearylgallat, Epigallocatechingallat sowie Gallussäure.

Beispielsweise lassen sich auch Extrakte des grünen Tees als pflanzliche Gerbstoffe einsetzen, ebenso Extrakte von Kastanien oder Mimosa.

Synthetische Gerbstoffe als solche sowie Verfahren zu deren Herstellung sind dem Fachmann bekannt. Geeignete synthetische Gerbstoffe, vorzugsweise mit einem M_{w}-Wert ≤ 3000 g/mol, sind beispielsweise in EP-A 0 301 406 oder DE-A 10 2005 050 193.1 offenbart. Methoden, wie durch Steuerung der Syntheseparameter die Molmasse in einem bestimmten Bereich gesteuert werden kann, sind dem Fachmann bekannt.

Vorzugsweise enthalten die erfindungsgemäßen Gemische als synthetischen Gerbstoff mindestens eines der nachfolgend aufgeführten Kondensationsprodukte (B) bis (D).

### Kondensationsprodukt (B)

Kondensationsprodukt (B) ist erhältlich durch Umsetzung von
b1) mindestens einem Aromaten oder Heteroaromaten,
b2) mindestens einer Carbonylverbindung,
b3) gegebenenfalls mindestens einem Sulfonierungsmittel und
b4) gegebenenfalls mindestens einem Harnstoffderivat.

Die Komponenten b1) bis b4) entsprechen inklusive der bevorzugten Definitionen den Komponenten a2) bis a5) des erfindungsgemäßen Kondensationsproduktes, wobei die Komponente b1) der Komponente a5) entspricht. Weiterhin ist im Gegensatz zu Komponente a5) außer Phenol auch Dihydroxydiphenylsulfon, insbesondere 4,4'-Dihydroxydiphenylsulfon, eine besonders bevorzugt Komponente b1).

In einer Ausführungsform der vorliegenden Erfindung weisen die Kondensationsprodukte (B) einen M_{w}-Wert ≤ 3000 g/mol auf. Verfahren zur Herstellung von Kondensationsprodukten (B) mit einem niedrigen M_{w}-Wert (M_{w}-Wert ≤ 3000 g/mol) sind dem Fachmann bekannt. Solche Kondensationsprodukte lassen sich gezielt insbesondere durch Beeinflussung von Parametern wie Reaktionszeit, Temperatur (eher niedriger), die Wahl der Monomere (beeinflusst die Reaktivität, insbesondere Verwendung von Dihydroxydiphenylsulfonen oder pH-Wert (schwach sauer) herstellen. Alternativ können Kondensationsprodukte (B) auch dadurch hergestellt werden, dass - wie für das erfindungsgemäße Kondensationsprodukt beschrieben - im Anschluss an die Synthese eines entsprechenden Kondensationsproduktes ein molekülgrößenabhängiges Trennverfahren, vorzugsweise eine Ultrafiltration, durchgeführt wird, wobei das Kondensationsprodukt (B) von allen übrigen Bestandteilen isoliert wird. Kondensationsprodukte (B) mit dem gewünschten M_{w}-Wert können insbesondere durch Verwendung eines Membrans mit einem geeigneten Molecular weight cut-off-Bereich von 1000 D - 2500D abgetrennt und isoliert werden.

### Kondensationsprodukt (C)

Kondensationsprodukt (C) ist erhältlich durch Umsetzung von
c1) Melamin und/oder Harnstoff,
c2) Glyoxal, Glyoxylsäure oder einem Alkalisalz davon,
c3) gegebenenfalls mindestens einer aromatische Verbindung mit mindestens einer phenolischen Hydroxylgruppe und
c4) gegebenenfalls mindestens einer kondensierbaren Verbindung mit einer reaktiven stickstoffhaltigen Gruppe.

Die Kondensationsprodukte (C) als solche sowie Verfahren zu deren Herstellung sind dem Fachmann bekannt. Beispielsweise werden diese in EP-A 0 301 406 beschrieben und sind unter Bezugnahme in die vorliegende Erfindung mit aufgenommen.

Als Komponente c3) eignen sich beispielsweise Phenolsulfonsäure, Sulfosalicylsäure, Salicylsäure und 8-Hydroxychinolin oder 4,4'-Dihydroxydiphenylsulfon. Als Komponente c4) eignen sich Carbonsäureamide, Sulfonsäureamide, Imide, Harnstoffe, Amino-und Iminosäuren sowie Dialkylamine und Dialkanolamine. Beispiele dafür sind Acetamid, Benzoesäureamid, Formamid, Amidosulfonsäure, Succinimid, Glycin, Iminodiessigsäure, Phenylglycin, Harnstoff, Dicyandiamid, Diethanolamin oder Diethylamin. Saure Verbindungen können dabei in Form ihrer Alkalisalze einkondensiert werden. Besonders bevorzugt ist als Komponente c4) Acetamid und Amidosulfonsäure.

Ein bevorzugtes Kondensationsprodukt (C) ist erhältlich durch Umsetzung von
c1) Melamin und/oder Harnstoff,
c2) Glyoxal und/oder Glyoxylsäure sowie
c4) gegebenenfalls Amidosulfonsäure.

### Kondensationsprodukt (D)

Kondensationsprodukt (D) ist erhältlich durch Umsetzung von
d1) mindestens einem cyclischen organischen Carbonat mit
d2) mindestens einer Verbindung mit mindestens zwei nucleophilen Gruppen pro Molekül, gewählt aus Sulfonsäure-, Hydroxyl-, primären oder sekundären Amino-oder Mercaptogruppen.

Kondensationsprodukte (D) als solche sowie Verfahren zu deren Herstellung sind dem Fachmann bekannt, sie sind beispielsweise in der deutschen Anmeldung mit der Nummer DE-A 10 2005 050 193.1 offenbart und unter Bezugnahme in die vorliegende Erfindung mit aufgenommen.

Unter cyclischen organischen Carbonaten (Komponente d1) versteht man im Rahmen der vorliegenden Erfindung organische Kohlensäureester, die mindestens eine cyclische Gruppe aufweisen.

Bevorzugt handelt es sich bei cyclischen organischen Carbonaten um solche organischen Kohlensäureester, bei denen die Kohlensäureestergruppe Bestandteil eines cyclischen Systems ist.

In einer Ausführungsform der vorliegenden Erfindung wählt man cyclisches organisches Carbonat (d1) aus Verbindungen der allgemeinen Formel (II) 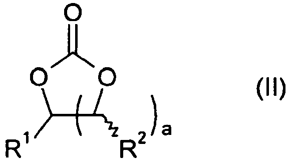 wobei die Variablen wie folgt definiert sind:
- R¹: gewählt aus C₁-C₄-Alkyl, verzweigt oder vorzugsweise linear, beispielsweise Methyl, Ethyl, n-Propyl, iso-Propyl, n-Butyl, iso-Butyl, sec-Butyl, tert.-Butyl, ganz bevorzugt Methyl und Ethyl, und ganz besonders bevorzugt Wasserstoff,
- R²: gegebenenfalls verschieden oder vorzugsweise gleich und unabhängig voneinander gewählt aus Wasserstoff und C₁-C₄-Alkyl, verzweigt oder vorzugsweise linear, beispielsweise Methyl, Ethyl, n-Propyl, iso-Propyl, n-Butyl, iso-Butyl, sec-Butyl, tert.-Butyl, ganz bevorzugt Methyl und Ethyl, und ganz besonders bevorzugt jeweils gleich und Wasserstoff,
a eine ganze Zahl im Bereich von 1 bis 3, bevorzugt 2 und besonders bevorzugt 1.

Besonders bevorzugte cyclische organische Carbonate d1) sind Propylencarbonat oder Ethylencarbonat. Mischungen von Propylencarbonat (R' = Methyl, R² = Wasserstoff, a = 1) und Ethylencarbonat (R' = R² = Wasserstoff, a = 1), insbesondere bei Zimmertemperatur flüssige Mischungen von Propylencarbonat und Ethylencarbonat sind ebenfalls besonders bevorzugt.

Unter Komponente d2) werden solche Verbindungen verstanden, die zwei zu nucleophilen Reaktionen befähigte Gruppen wie beispielsweise Sulfonsäuregruppen, Hydroxylgruppen, Mercaptogruppen oder primäre oder sekundäre Aminogruppen aufweisen.

Beispiele für geeignete Verbindungen d2) können aufweisen:
mindestens zwei nucleophile Hydroxylgruppen pro Molekül,
mindestens zwei nucleophile Mercaptogruppen pro Molekül,
mindestens zwei nucleophile primäre oder sekundäre Aminogruppen pro Molekül, beispielsweise zwei oder drei nucleophile primäre oder sekundäre Aminogruppen pro Molekül,
mindestens eine nucleophile Hydroxylgruppe oder Mercaptogruppe und mindestens eine nucleophile primäre oder sekundäre Aminogruppe pro Molekül oder
mindestens eine nucleophile Hydroxylgruppe und mindestens eine nucleophile Mercaptogruppe pro Molekül,
mindestens eine nucleophile Hydroxylgruppe oder primäre oder sekundäre Aminogruppe und eine Sulfonsäuregruppe pro Molekül.

Schwefelsäure ist keine Verbindung d2) im Sinne der vorliegenden Erfindung.

Beispiele für nucleophile Hydroxylgruppen sind OH-Gruppen von primären und sekundären Alkoholen und insbesondere phenolische OH-Gruppen.
Beispiele für nucleophile Mercaptogruppen sind SH-Gruppen, äliphatisch oder aromatisch.

Beispiele für nucleophile Aminogruppen sind -NHR³-Gruppen, aliphatisch oder aromatisch, wobei R³ gewählt wird aus Wasserstoff, C₁-C₄-Alkyl, wie vorstehend definiert, und CN, oder die NH₂-Gruppe von beispielsweise Amidosulfonsäure.

OH-Gruppen und NH-Gruppen, die Bestandteile von Aminalgruppen, Halbaminalgruppen oder Hydratgruppen von Ketonen oder Aldehyden sind, sind keine nucleophilen Hydroxylgruppen beziehungsweise Aminogruppen im Sinne der vorliegenden Erfindung. Auch OH-Gruppen und NH-Gruppen, die Bestandteile von Carbonsäuregruppen oder Carbonsäureamidgruppen sind, sind keine nucleophilen Hydroxylgruppen beziehungsweise Aminogruppen im Sinne der vorliegenden Erfindung.

Bevorzugte Beispiele für Verbindungen d2) sind
i) Harnstoffe, unsubstituiert oder ein- oder zweifach N,N'-substituiert mit C₁-C₄-Alkyl, Biuret, insbesondere unsubstituierter Harnstoff,
ii) heterocyclische Verbindungen mit mindestens zwei NH₂-Gruppen pro Molekül, beispielsweise Adenin und insbesondere Melamin,
iii) Benzoguanamin, Dicyandiamid, Guanidin,
iv) Verbindungen der allgemeinen Formel (III) 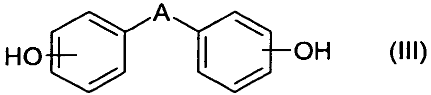 in denen A eine bivalente Gruppe bedeutet, beispielsweise -CH₂-, -CH₂CH₂, -CH(CH₃)-, -C(CH₃)₂-, -CO-, -SO₂-, bevorzugt 4,4'-Dihydroxybiphenyl, 2,4'-Dihydroxydiphenylsulfon, besonders bevorzugt 4,4'-Dihydroxydiphenylsulfon, Mischungen von 4,4'-Dihydroxydiphenylsulfon und 2,4'-Dihydroxydiphenylsulfon, beispielsweise in einem Gewichtsverhältnis von 8 : 1 bis 8 : 1,5, sowie Bisphenol A.

Weitere bevorzugte Beispiele für Verbindung d2) sind 4-Hydroxyphenylsulfonsäure und Amidosulfonsäure.

Besonders bevorzugte Verbindungen d2) sind ausgewählt aus Melamin, Biuret, Dicyanamid, Amidosulfonsäure und 4,4'Dihydroxydiphenylsulfon.

In einer Ausführungsform der vorliegenden Erfindung werden Gemische eingesetzt, in denen mindestens ein erfindungsgemäßes Kondensationsprodukt und/oder mindestens ein synthetischer Gerbstoff mit einem M_{w}-Wert ≤ 3000 g/mol hergestellt werden unter Verwendung von mindestens einer Verbindung, die mindestens eine Hydroxylgruppe enthält beziehungsweise mit einer solchen Gruppe substituiert ist. Vorzugsweise wird dies erzielt, indem
die Komponente a5) mindestens eine Verbindung enthält, die mit mindestens einer Hydroxylgruppe substituiert ist und/oder
die Komponente b1) mindestens eine Verbindung enthält, die mit mindestens einer Hydroxylgruppe substituiert ist und/oder
die Komponente c3) vorhanden ist und/oder
die Komponente d2) mindestens eine Verbindung enthält mit mindestens einer Hydroxylgruppe als nucleophiler Gruppe.

In einer weiteren bevorzugten Ausführungsform der vorliegenden Erfindung werden Gemische eingesetzt, bei denen der weitere Gerbstoff formaldehydfrei ist, vorzugsweise ein synthetischer formaldehydfreier Gerbstoff. Vorzugsweise wird dies erzielt, indem ein Kondensationsprodukt (C) oder Kondensationsprodukt (D) im Gemisch eingesetzt wird.

Gegenstand der vorliegenden Erfindung ist weiterhin die Verwendung der erfindungsgemäßen Kondensationsprodukte, gegebenenfalls im Rahmen eines der vorstehend beschriebenen Gemische, als Arzneimittel beziehungsweise Medikament.

Die erfindungsgemäßen Kondensationsprodukte eignen sich insbesondere als antivirales Agens, also als Arzneimittel gegen Viren, auch als Virustatika oder virucide Mittel bezeichnet. Vorzugsweise eignen sie sich als antivirales Agens gegenüber humanen Papillomviren, speziell Typ 16, 18, 6 und 11, endogenen Retroviren, insbesondere HERV-Typ (Humane endogene Retroviren), Herpes-Viren, insbesondere HSV-1, HCMV-Viren (Humanes Zytomegalievirus) oder HIV-Viren.

Weiterhin eignen sich die erfindungsgemäßen Kondensationsprodukte vorzugsweise als antivirales Agens gegenüber Coronaviren (z.B. SARS (Severe Acute Respiratory Syndrome)-assoziiertes Coronavirus), Flaviviren (z.B. West-Nil-Virus (WNV)), Togaviren (z.B. Chikungunyavirus) oder Paramoxyviren (z.B. Masern, Respiratorisches Syncytialvirus (RSV).

Weiterhin eignen sich die erfindungsgemäßen Kondensationsprodukte vorzugsweise zur Inhibierung von Serin Proteasen. Serin Proteasen umfassen die Humane Leukozyten Elastase (HLE; auch als Humane Neutrophile Elastase (HNE) bezeichnet), Proteinase 3 und Cathepsin G. Vorzugsweise handelt es sich bei den Serin Proteasen um HLE oder die Proteinase 3, insbesondere um HLE.

Vorzugsweise eignen sich die erfindungsgemäßen Kondensationsprodukte (zur Herstellung von einem Medikament) zur Prophylaxe und/oder Behandlung von Genitalwarzen, Gebärmutterhalskrebs, allergischen oder nicht-allergischen Ekzemen, insbesondere Neurodermitis (endogenes Ekzem), Wundsein, Juckreiz, entzündlichen Erkrankungen, Autoimmunerkrankungen, insbesondere Arthritis, Rheuma, von melanomen Karzinomen, Entzündungen der Haut, Herpes, insbesondere Herpes labilis und Herpes simplex, Windpocken, Gürtelrose, Influenza oder Aids (HIV).

Weiterhin eignen sich die erfindungsgemäßen Kondensationsprodukte zur Prophylaxe und/oder Behandlung von Lungenerkrankungen, Myokard-Perfusion, Ischämieschäden des Hirns, Peritonitis, septischem Schock oder systemischen Vaskulitiden. Bei den Lungenerkrankungen handelt es sich vorzugsweise um chronisch obstruktive Lungenerkrankungen (COPD). Vorzugsweise wird bei den Lungenerkrankungen das erfindungsgemäße Kondensationsprodukt in Form eines Aerosols verabreicht. Bei den systemischen Vaskulitiden handelt handelt es sich vorzugsweise um die Wegener'sche Granulomatose.

Weiterhin eignen sich die erfindungsgemäßen Kondensationsprodukte zur Prophylaxe und/oder Behandlung von bullösem Pemphigoid, Psoriasis vulgaris, der allergenen Potenz von Typ 1-Allergenen, allergischer Rhinitis, allergischer Konjunktivitis, allergischem Asthma, Bronchiale oder Schuppenflechte.

In einer Ausführungsform der vorliegenden Erfindung handelt es sich bei Arzneimitteln um Arzneimittel zur lokalen Behandlung von allergischen oder nicht-allergischen Ekzemen, Wundsein oder Juckreiz. Vorzugsweise wird in dieser Ausführungsform Neurodermitis (endogenes Ekzem) behandelt.

In einer speziellen Ausführungsform der vorliegenden Erfindung handelt es sich bei Arzneimitteln um Arzneimittel zur Behandlung von entzündlichen Erkrankungen der Haut, bei denen es durch enzymatische Aktivität z.B. der humanen Leukozyten-Elastase zur Bildung von Bläschen, Pusteln und zur so genannten Spongiose in der Oberhaut kommt. Die Arzneimittel werden bevorzugt äußerlich appliziert

In einer bevorzugten Ausführungsform der vorliegenden Erfindung handelt es sich bei Arzneimitteln um Arzneimittel gegen Viren, vorzugsweise Retroviren, beispielsweise RNS-Viren (Ribonucleinsäure-Viren) und DNS-Viren (Desoxyribonucleinsäure-Viren) und insbesondere Herpes-Viren, beispielsweise Viren, die Herpes simplex (HS-Viren) erzeugen, oder auch um Viren, die Windpocken und Influenza erzeugen. Ferner ist anzumerken, dass die erfindungsgemäßen Wirkstoffe sowohl gegen hydrophile als auch ebenso wirksam gegen lipophile/hydrophobe Viren eingesetzt werden können.

In einer weiteren Ausführungsform der vorliegenden Erfindung handelt es sich bei Arzneimitteln um Arzneimittel gegen HIV-Viren (Human Imuno Deficiency Virus). Der HIV-Virus ist dafür bekannt, dass er Aids (Aquired Imuno Deficiency Syndrom) verursacht.

In einer weiteren bevorzugten Ausführungsform der vorliegenden Erfindung handelt es sich bei Arzneimitteln um Arzneimittel gegen Humane Papillomviren sowie von endogenen Retroviren (HERV-Typ). Bei den Human-Papillomviren handelt es sich insbesondere um den Typ 16, 18, 6 und 11. In dieser Hinsicht eignen sich die erfindungsgemäßen Kondensationsprodukte insbesondere zur äußerlichen Medikation von Genitalwarzen und Gebärmutterhalskrebs. Im Zusammenhang mit der Behandlung von HERV-Viren (insbesondere HERV-K) eignen sich die erfindungsgemäßen Kondensationsprodukt zur Behandlung von Autoimmunkrankheiten (Arthritis) sowie präventiv gegen melanome Karzinome.

In den vorstehenden Ausführungen umfasst der Begriff Behandlung auch die Prophylaxe, Therapie oder Heilung der vorgenannten Krankheiten.

Die erfindungsgemäßen Kondensationsprodukte können Tieren und Menschen, bevorzugt Säugetieren und Menschen, besonders bevorzugt Menschen, verabreicht werden. Die erfindungsgemäßen Kondensationsprodukte können dabei selbst als Arzneimittel, in Mischungen miteinander oder Mischungen mit anderen Arzneimitteln oder in Form von pharmazeutischen Zusammensetzungen verabreicht werden. Folglich sind die Verwendung von den erfindungsgemäßen Kondensationsprodukten zur Herstellung eines oder mehrerer Medikamente zur Prophylaxe und/oder Behandlung der vorgenannten Krankheiten oder als antivirales Agens, pharmazeutische Zusammensetzungen enthaltend eine wirksame Menge von mindestens einem erfindungsgemäßen Kondensationsprodukt sowie der Verwendung dieser pharmazeutischen Zusammensetzungen zur Prophylaxe und/oder Behandlung der vorgenannten Krankheiten ebenfalls Gegenstand der vorliegenden Erfindung.

Die erfindungsgemäßen pharmazeutischen Zusammensetzungen umfassen eine wirksame Menge von mindestens einem erfindungsgemäßen Kondensationsprodukt sowie einen physiologisch verträglichen Träger. Die pharmazeutischen Zusammensetzungen können dabei in unterschiedlichen Darreichungsformen vorliegen, insbesondere in Form einer Pille, Tablette, Lutschtablette, Granulat, Kapsel, harte oder weiche Gelatinekapsel, wässrigen Lösung, alkoholischen Lösung, ölartigen Lösung, Sirup, Emulsion, Suspension, Zäpfchen, Pastille, Lösung zur Injektion oder Infusion, Salbe, Tinktur, Creme, Lotion, Puder, Spray, eines transdermalen therapeutischen Systems, Nasenspray, Aerosol, Aerosol-Mischung, Mikrokapsel, Implantat, Stab, Pflaster oder Gel. Ebenso kann die erfindungsgemäße pharmazeutische Zusammensetzung auch Bestandteil von Health-Care Produkten wie Sonnenschutzcremes, Nasensprays, Mundwässern, Zahnpasten, Pflastern, (Feucht)-Tüchern, Waschlotionen oder Shampoos sein.

Je nach verwendeter Darreichungsform werden die erfindungsgemäßen Kondensationsprodukte mit physiologisch verträglichen Trägern, die dem Fachmann als solche bekannt sind, zu den erfindungsgemäßen pharmazeutischen Zusammensetzungen verarbeitet. Der Träger muss natürlich verträglich sein in dem Sinn, dass er mit den anderen Bestandteilen der Zusammensetzung kompatibel ist und nicht gesundheitsschädlich für den Patienten ist (physiologisch verträglich). Der Träger kann ein Feststoff oder eine Flüssigkeit oder beides sein und wird vorzugsweise mit der Verbindung als Einzeldosis formuliert, beispielsweise als Tablette, die von 0,05 bis 95 Gew.-% des Wirkstoffs (erfindungsgemäßes Kondensationsprodukt) enthalten kann. Weitere pharmazeutisch aktive Substanzen können ebenfalls vorhanden sein. Die erfindungsgemäßen pharmazeutischen Zusammensetzungen können nach einer der bekannten pharmazeutischen Methoden hergestellt werden, die im Wesentlichen darin besteht, dass die Bestandteile mit pharmakologisch verträglichen Trägern und/oder weiteren Hilfsstoffen wie Füllstoffe, Bindemittel, Gleitmittel, Netzmittel, Stabilisatoren und so weiter gemischt werden.

Bevorzugte pharmazeutische Zusammensetzungen im Rahmen der vorliegenden Erfindung sind nachfolgend aufgeführt.

In einer Ausführungsform der vorliegenden Erfindung können erfindungsgemäße Salben, Cremes, Fettcremes, Gele, Lotionen beziehungsweise Puder jeweils im Bereich von 0,1 bis 5 Gew.%, bevorzugt 0,2 bis 3 Gew.-% erfindungsgemäße Kondensationsprodukte enthalten, bezogen auf die jeweilige Salbe, Creme, Fettcreme, Lotion beziehungsweise das jeweilige Gel oder Puder.

In einer Ausführungsform der vorliegenden Erfindung können erfindungsgemäße Pulver beziehungsweise-Konzentrate im Bereich von 1 bis 75 Gew.%, bevorzugt 10 bis 65 Gew.-% erfindungsgemäßes Kondensationsprodukt enthalten, bezogen auf das jeweilige Pulver beziehungsweise Konzentrat.

Erfindungsgemäße Cremes sind üblicherweise Öl-in-Wasser-Emulsionen, erfindungsgemäße Salben sind üblicherweise Wasser-in-Öl-Emulsionen. Erfindungsgemäße Salben und Cremes enthalten neben vorzugsweise gereinigtem Wasser eine oder mehrere Ölkomponenten und vorzugsweise eine oder mehrere oberflächenaktive Substanzen, beispielsweise einen oder mehrere Emulgatoren oder Schutzkolloide. Weiterhin können erfindungsgemäße Salben und Fettcremes - wie andere erfindungsgemäße Darreichungsformen der erfindungsgemäßen Kondensationsprodukte auch - Konservierungsmittel enthalten wie beispielsweise Sorbinsäure.

Geeignete Ölkomponenten sind natürliche und synthetische Wachse, natürliche und synthetische Öle wie beispielsweise Nussöl, Fischöl, Olivenöl und Polymere wie beispielsweise Polyacrylsäure, Polydimethylsiloxan und Polymethylphenylsiloxan.

Geeignete oberflächenaktive Substanzen sind beispielsweise Verbindungen der allgemeinen Formel (IV)

CH₃-(CH₂)ₙ-X-R³ (IV)

wobei die Variablen wie folgt definiert sind:
n ist eine ganze Zahl im Bereich von 0 bis 20, bevorzugt eine gerade Zahl im Bereich von 2 bis 16 bedeutet und
X steht für zweibindige Gruppen, die mindestens ein von Kohlenstoff und Wasserstoff verschiedenes Atom, bevorzugt Stickstoff und besonders bevorzugt Sauerstoff tragen, insbesondere -O- und -COO-,
R³ wird gewählt aus
Wasserstoff,
C₁-C₁₀₋Alkylgruppen wie beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl; besonders bevorzugt C₁-C₄-Alkyl wie Methyl, Ethyl, n-Propyl, iso-Propyl, n-Butyl, iso-Butyl, sec.-Butyl und tert.-Butyl,
-(CH₂-CH₂-O)ₘ-H, wobei m eine ganze Zahl im Bereich von 1 bis 100, bevorzugt bis 25 ist,
CH₃-(CH₂)ₙ-X-(O-CH₂-CH₂)ₘ-, wobei X und n jeweils verschieden oder vorzugsweise gleich sein können.

Weiterhin können erfindungsgemäße Salben und Cremes - wie andere erfindungsgemäße Darreichungsformen der erfindungsgemäßen Kondensationsprodukte auch - organische Lösungsmittel enthalten wie beispielsweise Propylenglykol und Glycerin.

Bevorzugte Beispiele für oberflächenaktive Substanzen sind beispielsweise Isopropyltetradecanoat, Cetylalkohol, Palmitinsäure, Stearinsäure, Polyoxyethylen-2-stearylether, α-n-Dodecyl-ω-hydroxypolyoxyethylen mit im Mittel 10 Ethylenoxideinheiten, 2-Phenoxyethanol, Polyoxyethylen-21-stearylether.

Erfindungsgemäße Fettcremes sind üblicherweise Wasser-in-Öl-Emulsionen und enthalten neben vorzugsweise gereinigtem Wasser ein oder mehrere Ölkomponenten und vorzugsweise eine oder mehrere oberflächenaktive Substanzen, beispielsweise einen oder mehrere Emulgatoren oder Schutzkolloide.

Geeignete Ölkomponenten sind neben den vorstehend beschriebenen Ölkomponenten natürliche und synthetische Fette wie beispielsweise ein- oder mehrfach ethylenisch ungesättigte Fettsäureglyceride.

Weiterhin können erfindungsgemäße Fettcremes eine oder mehrere der folgenden Substanzen enthalten: Methyl-4-hydroxybenzoat, Propyl-4-hydroxybenzoat, wässrige Sorbit-Lösung, Tris[n-dodecylpoly(oxoethylen)-4]phosphat, Cetylstearylalkohol, Hexyllaurat, Vitamin-F-Glycerolester, Dimeticon 350, Calciumlactat-Pentahydrat.

Erfindungsgemäße Gele können beispielsweise Polyacrylsäure, Natriumhydroxid und Butylhydroxyanisol, beispielsweise 4-Methoxy-2-tert.-butylphenol, 4-Methoxy-3-tert.-butylphenol und Mischungen der beiden vorgenannten Verbindungen enthalten.

Erfindungsgemäße Lotionen können beispielsweise mindestens einen der im Folgenden genannten Stoffe enthalten: Glycerin, Zinkoxid, Talkum, Lecithin, hochdisperses Siliciumdioxid, Isopropanol, Methyl(4-hydroxybenzoat), Carageenan, Natriumsalz und Phosphorsäureester der allgemeinen Formel (V) 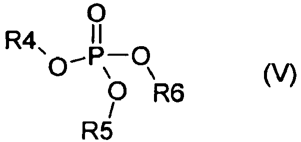 in denen R⁴, R⁵ und R⁶ gleich oder verschieden sein können und gewählt werden aus n-C₁₀-C₂₀-Alkyl, insbesondere n-C₁₆-C₁₈-Alkyl und H-(O-CH₂-CH₂)ₘ, wobei m wie oben stehend definiert ist.

Erfindungsgemäße Puder können beispielsweise enthalten: Calciumlactat-Pentahydrat, Talkum, Maisstärke, 2-n-Octyl-1-dodecanol, Siliciumdioxid.

Erfindungsgemäße Pulver zur Herstellung von Gebrauchslösungen können beispielsweise Calciumlactat 5 H₂O und Natriumsulfat (als Trägermaterial) enthalten.

Erfindungsgemäße Konzentrate zur Herstellung von Gebrauchslösungen können beispielsweise enthalten: Natriumsalz des Dodecylpoly(oxyethylen)-2-hydrogensulfats, Natriumsulfat als Trägermaterial.

Anstatt erfindungsgemäße Salben, Cremes, Fettcremes, Gele, Lotionen, Puder; Pulver oder Konzentrate auf ihre Wirksamkeit zu untersuchen, kann man erfindungsgemäße Kondensationsprodukte, gegebenenfalls als Stammlösung, auf ihre Wirksamkeit untersuchen. Geeignete Untersuchungsmethoden sind Untersuchungen auf Hemmung von ausgesuchten Enzymen, beispielsweise humane Leukozyten-Elastase oder der Protease Plasmin. Weiterhin kann man untersuchen, in welchem Maße die Replikation betreffender Viren gehemmt wird. Solche Untersuchungsmethoden werden im nachfolgend Text (Pharmakalogische Untersuchungen) noch genauer beschrieben.

Ein weiterer Gegenstand der vorliegenden Erfindung ist die Verwendung eines erfindungsgemäßen Kondensationsproduktes zur Desinfektion, als Desinfektionsmittel oder Bestandteil eines Desinfektionsmittels. Insbesondere finden die erfindungsgemäßen Kondensationsprodukte Verwendung im Bereich von Krankenhäusern, insbesondere Krankenhaus-Intensivstationen, Toiletten, Waschräumen, Haushalten, der Lebensmittelproduktion oder in Stallungen oder Käfigen von Tieren, insbesondere von Vögeln, Schweinen und Rindern.

Die erfindungsgemäßen Kondensationsprodukte zeichnen sich bei ihrer Verwendung als Desinfektionsmittel dadurch aus, dass sie eine überraschend gute Breitbandwirkung gegenüber Pilzen, Bakterien und Viren haben sowie eine geringere Toxizität gegenüber den üblichen Mitteln beziehungsweise Mischungen, die als Desinfektionsmittel gemäß des Standes der Technik verwendet werden, aufweisen. Weiterhin sind sie weder flüchtig noch schleimhautreizend und sie sind sowohl als flüssige oder auch streufähige Pulvereinstellung leicht herstellbar. Insbesondere eignen sich die erfindungsgemäßen Kondensationsprodukte zur Anwendung in Stallungen oder Käfigen von Tieren, vorzugsweise auf Stroh.

Ein weiterer Gegenstand der vorliegenden Erfindung ist somit auch ein Desinfektionsmittel enthaltend mindestens ein erfindungsgemäßes Kondensationsprodukt

Die erfindungsgemäßen Desinfektionsmittel sind somit nicht zur Verabreichung als Arzneimittel gedacht, sondern sie sind zur Desinfektion von beispielsweise den vorstehend aufgeführten Gegenständen geeignet. In den erfindungsgemäßen Desinfektionsmitteln ist mindestens ein erfindungsgemäßes Kondensationsprodukt in den üblichen Konzentrationen enthalten. Weitere Komponenten, die in den erfindungsgemäßen Desinfektionsmitteln enthalten sind, sind dem Fachmann bekannt. Solche Komponenten können je nach Anwendungsgebiet variieren, gleiches gilt für die Konzentration an dem erfindungsgemäßen Kondensationsprodukt.

Ein weiterer Gegenstand der vorliegenden Erfindung ist die Verwendung der erfindungsgemäßen Kondensationsprodukte als Gerbstoff bzw. als Gerbereihilfsmittel. Vorzugsweise eignen sich die erfindungsgemäßen Kondensationsprodukte zum Gerben von Ledern und/oder Häuten. Verfahren zum Gerben (Gerbverfahren) als solche sind dem Fachmann bekannt, sie beispielsweise EP-A 0 301 406.

Die Erfindung soll an den nachfolgenden Beispielen verdeutlicht werden.

### Beispiele

### Allgemeine Angaben

ppm beziehen sich stets auf Gewichtsanteile, sofern nicht anders angegeben.
Die Molekulargewichtsbestimmungen werden mit Gelpermeationschromatographie (GPC) durchgeführt:
Stationäre Phase: mit Ethylenglykoldimethacrylat vernetztes Poly-(2-hydroxymeth-acrylat)-Gel, kommerziell erhältlich als HEMA BIO von Fa. PSS, Mainz, Deutschland. Laufmittel: Gemisch aus 30 Gew.% Tetrahydrofuran (THF), 10 Gew.% Acrylnitril, 60 Gew.-% 1-molare NaNO₃-Lösung
Interner Standard: 0,001 Gew.-% Benzophenon, bezogen auf Laufmittel
Fluss: 1,5 ml/min
Konzentration: 1 Gew.-% im Laufmittel mit internem Standard
Detektion: UV/Vis-spektrometrisch bei 254 nm
Kalibrierung mit Polystyrol-Eichteils der Fa. PSS.
Mₙ: zahlenmittleres Molekulargewicht in [g/mol]
M_{w}: gewichtsmitteleres Molekulargewicht in [g/mol]

### 1. Synthesebeispiele von Reaktionsgemischen

### Beispiel 1

Reaktanden (Edukte):
a) Phenol,
b) konzentrierte Schwefelsäure,
c) Harnstoff
d) Formaldehyd
e) 1-Naphtalincarbonsäure

### Vorgehen:

47,1 g Phenol werden in einer Rührapparatur vorgelegt und während 25 Minuten mit 57,1 g konzentrierter Schwefelsäure (96 Gew.-%) versetzt. Dabei wird darauf geachtet, dass die Temperatur 105°C nicht übersteigt. Anschließend wird das Reaktionsgemisch 2 Stunden bei 100 bis 105°C gerührt. Das Reaktionsgemisch wird dann mit 7,9 g Wasser verdünnt. Es werden 64,6 g wässrige Harnstofflösung (50 Gew.-%) zudosiert, wobei die Temperatur auf 95°C steigt; anschließend wird auf 75°C gekühlt. Über einen Zeitraum von 90 Minuten werden 94,6 g wässrige Formaldehydlösung (30 Gew.-%) zugefügt, wobei darauf geachtet wird, dass die Temperatur nicht über 75°C steigt. Anschließend wird mit 17,3 g wässriger Natronlauge (50 Gew.-%) teilneutralisiert, und 6,9 g Wasser wird zugefügt. Bei einer Temperatur von 50 bis 55°C werden 57,2 g 1-Naphtalincarbonsäure und 0,6 g eines Komplexbildners auf Basis Etyhlendiamin - Tetraessigsäure zugegeben. Das Reaktionsgemisch wird dann 30 Minuten bei 55°C nachgerührt, bevor 26,3 g wässriger Formaldehydlösung (30 Gew.-%) innerhalb von 15 bis 20 Minuten bei 55 bis 60°C zudosiert werden. 74,4 g wässriger Natronlauge (50 Gew.-%) und 46,2 g Wasser werden zugegeben. Nach Verdampfung der flüchtigen Anteile erhält man 152,5 g Kondensationsprodukt 1 als Feststoff mit weißer Farbe.

Die Analyse des Reaktionsgemisches 1 durch HPLC ergibt folgende Werte:
Phenol: < 0,1 Gew.%;
4-Phenolsulfonsäure: 9,2 Gew.-%;
freier Formaldehyd: < 20 ppm;
Mₙ 2390 g/mol, M_{w} 35630 g/mol, bestimmt durch GPC.

### Beispiel 2

Reaktanden:
a) Phenol,
b) konzentrierte Schwefelsäure,
c) Harnstoff,
d) Formaldehyd,
e) 1-Naphtalincarbonsäure

### Vorgehen:

47,1 g Phenol werden in einer Rührapparatur vorgelegt und während 25 Minuten mit 57,1 g konzentrierter Schwefelsäure (96 Gew.-%) versetzt. Dabei wird darauf geachtet, dass die Temperatur 105°C nicht übersteigt. Anschließend wird das Reaktionsgemisch 2 Stunden bei 100 bis 105°C gerührt. Das Reaktionsgemisch wird dann mit 8 g Wasser verdünnt. Es werden 64,6 g wässrige Harnstofflösung (50 Gew.-%) zudosiert, wobei die Temperatur auf 95°C steigt; anschließend wird auf 75°C gekühlt. Über einen Zeitraum von 90 Minuten werden 141,9 g wässrige Formaldehydlösung (30 Gew.-%) zugefügt, wobei darauf geachtet wird, dass die Temperatur nicht über 75°C steigt. Anschließend wird mit 17,3 g wässriger Natronlauge (50 Gew.-%) teilneutralisiert, und 10 g Wasser wird zugefügt. Bei einer Temperatur von 50 bis 55°C werden 57,2 g 1-Naphtalincarbonsäure und 0,6 g eines Komplexbildners auf Basis Etyhlendiamin-Tetraessigsäure zugegeben. Das Reaktionsgemisch wird dann 30 Minuten bei 55°C nachgerührt, bevor 26,3 g wässriger Formaldehydlösung (30 Gew.-%) innerhalb von 15 bis 20 Minuten bei 55 bis 60°C zudosiert werden. Wässrige Natronlauge (50 Gew.-%) und 100 g Wasser werden bis zum einem pH-Wert von 7,2 zugegeben. Nach Verdampfung der flüchtigen Anteile erhält man das Kondensationsprodukt 2.

### Vergleichsbeispiel 3

Reaktanden:
a) 1-Naphtalincarbonsäure,
b) Harnstoff,
c) Formaldehyd,

### Vorgehen:

47,7 g 1-Naphtalincarbonsäure werden in einer Rührapparatur in 250 g Wasser und 22,5 g wässrige Natronlauge (50 Gew.-%) gelöst. Es wird darauf geachtet, dass der pH-Wert dieser wässrigen Lösung nicht über 9 steigt. Es werden 64,6 g wässrige Harnstofflösung (50 Gew.-%) bei 70°C zudosiert, wobei die Temperatur auf 95°C steigt; anschließend wird auf 75°C gekühlt. Über einen Zeitraum von 90 Minuten werden 94,6 g wässrige Formaldehydlösung (30 Gew.-%) zugefügt, wobei darauf geachtet wird, dass die Temperatur nicht über 75°C steigt. Bei einer Temperatur von 50 bis 55°C werden 57,2 g 1-Naphtalincarbonsäure und 0,6 g eines Komplexbildners auf Basis Etyhlendiamin - Tetraessigsäure zugegeben. Das Reaktionsgemisch wird dann 30 Minuten bei 55°C nachgerührt, bevor 26,3 g wässriger Formaldehydlösung (30 Gew.-%) innerhalb von 15 bis 20 Minuten bei 8°C zudosiert werden. 26,5 g wässriger Natronlauge (50 Gew.-%) werden zugegeben. Nach Verdampfung der flüchtigen Anteile erhält man 158,2 g Kondensationsprodukt 3 als Pulver mit weißer Farbe.

### Vergleichsbeispiel 4

Reaktanden:
a) Phenol,
b) konzentrierte Schwefelsäure,
c) Harnstoff,
d) Formaldehyd,
d) Naphtalinsulfonsäure

### Vorgehen:

47,1 g Phenol werden in einer Rührapparatur vorgelegt und während 25 Minuten mit 57,1 g konzentrierter Schwefelsäure (96 Gew.-%) versetzt. Dabei wird darauf geachtet, dass die Temperatur 105°C nicht übersteigt. Anschließend wird das Reaktionsgemisch 2 Stunden bei 100 bis 105°C gerührt. Das Reaktionsgemisch wird dann mit 7,9 g Wasser verdünnt. Es werden 64,6 g wässrige Harnstofflösung (50 Gew.-%) zudosiert, wobei die Temperatur auf 95°C steigt; anschließend wird auf 75°C gekühlt. Über einen Zeitraum von 90 Minuten werden 94,6 g wässrige Formaldehydlösung (30 Gew.-%) zugefügt, wobei darauf geachtet wird, dass die Temperatur nicht über 75°C steigt. Bei einer Temperatur von 50 bis 55°C werden 69,3 g Naphtalinsulfonsäure und 0,6 g eines Komplexbildners auf Basis Etyhlendiamin - Tetraessigsäure zugegeben. Das Reaktionsgemisch wird dann 30 Minuten bei 55°C nachgerührt, bevor 26,3 g wässriger Formaldehydlösung (30 Gew.-%) innerhalb von 15 bis 20 Minuten bei 55 bis 60°C zudosiert werden. Wässrige Natronlauge (50 Gew.-%) werden bis zum einem pH-Wert von 7,2 zugegeben. Nach Verdampfung der flüchtigen Anteile erhält man das Kondensationsprodukt 4.

### Vergleichsbeispiel 5

Reaktanden:
a) Phenol,
b) konzentrierte Schwefelsäure,
c) Formaldehyd,
d) 1-Naphtalincarbonsäure

### Vorgehen:

47,1 g Phenol werden in einer Rührapparatur vorgelegt und während 25 Minuten mit 57,1 g konzentrierter Schwefelsäure (96 Gew.-%) versetzt. Dabei wird darauf geachtet, dass die Temperatur 105°C nicht übersteigt. Anschließend wird das Reaktionsgemisch 2 Stunden bei 100 bis 105°C gerührt. Das Reaktionsgemisch wird dann mit 7,9 g Wasser verdünnt. Über einen Zeitraum von 90 Minuten werden bei 75°C 94,6 g wässrige Formaldehydlösung (30 Gew.-%) zugefügt, wobei darauf geachtet wird, dass die Temperatur nicht über 75°C steigt. Bei einer Temperatur von 50 bis 55°C werden 57,2 g 1-Naphtalincarbonsäure und 0,6 g eines Komplexbildners auf Basis Etyhlendiamin - Tetraessigsäure zugegeben. Das Reaktionsgemisch wird dann 30 Minuten bei 55°C nachgerührt, bevor 26,3 g wässriger Formaldehydlösung (30 Gew.-%) innerhalb von 15 bis 20 Minuten bei 55 bis 60°C zudosiert werden. Wässrige Natronlauge (50 Gew.-%) werden bis zum einem pH-Wert von 7,2 zugegeben. Nach Verdampfung der flüchtigen Anteile erhält man das Kondensationsprodukt 5.

### Vergleichsbeispiel 6

Reaktanden:
a) Phenol,
b) konzentrierte Schwefelsäure,
c) Harnstoff
d) Formaldehyd

### Vorgehen:

20,4 g Phenol werden in einer Rührapparatur vorgelegt und während 25 Minuten mit 24,7 g konzentrierter Schwefelsäure (96 Gew.-%) versetzt. Dabei wird darauf geachtet, dass die Temperatur 105°C nicht übersteigt. Anschließend wird das Reaktionsgemisch 2 Stunden bei 100 bis 105°C gerührt. Das Reaktionsgemisch wird dann mit 3,4 g Wasser verdünnt. Es werden 28,1 g wässrige Harnstofflösung (50 Gew.-%) zudosiert, wobei die Temperatur auf 95°C steigt; anschließend wird auf 75°C gekühlt. Über einen Zeitraum von 90 Minuten werden 41,0 g wässrige Formaldehydlösung (30 Gew.-%) zugefügt, wobei darauf geachtet wird, dass die Temperatur nicht über 75°C steigt. Anschließend wird mit 7,5 g wässriger Natronlauge (50 Gew.-%) teilneutralisiert, und 3 g Wasser wird zugefügt.
Bei einer Temperatur von 50 bis 55°C werden 13,6 g Phenol und 0,3 g eines Komplexbildners auf Basis Etyhlendiamin - Tetraessigsäure zugegeben. Das Reaktionsgemisch wird dann 30 Minuten bei 55°C nachgerührt, bevor 11,4 g wässriger Formaldehydlösung (30 Gew.-%) innerhalb von 15 bis 20 Minuten bei 55 bis 60°C zudosiert werden. 32,2 g wässriger Natronlauge (50 Gew.-%) und 20 g Wasser werden zugegeben. Die Endeinstellung vom pH-Wert bis pH 7,0 - 7,4 erfolgt durch Zugabe von 8,2 g Schwefelsäure (50 Gew.-%). Nach Verdampfung der flüchtigen Anteile erhält man 200 g Reaktionsgemisch 6 als farblosen Feststoff.

Die Analyse des Kondensationsproduktes 6 ergibt folgende Werte:
Phenol durch HPLC: < 0,1 Gew.-%;
4-Phenolsulfonsäure durch HPLC: 7,8 Gew.-%;
freier Formaldehyd: < 20 ppm;

### 2. Pharmakologische Untersuchungen

### Verfahren zur Bestimmung der antiviralen Aktivität

In der Untersuchung wird bestimmt, ob ein Kondensationsprodukt antivirale Aktivität gegen verschiedene Viren aufweist und welche Menge antiviraler Substanz benötigt wird, um eine 50% Reduktion der Virusreplikation zu bewirken.

Die Virusgebrauchsverdünnung wird an Hand einer Endpunkttitration des angezüchteten Virusisolates ermittelt. Bei dieser Titration wird die Virusmenge ermittelt, bei der 50% der Ansätze aus der Virusverdünnung infiziert bzw. nicht infiziert sind (= infektiöse Dosis 50% = TCID₅₀/ml).

Aus der zu testenden Substanz wird eine um Faktor zwei ansteigende Verdünnungsreihe hergestellt. Danach wird eine definierte Menge Virus zugegeben.

Das Stoff/Virus-Gemisch wird auf Monolayer aus geeigneten Zellen gegeben. Nach einer vom Virus abhängigen Inkubationszeit erfolgt eine Beurteilung der virusbedingten zytopathogenen Veränderung (CPE). Zur Ermittlung der Ergebnisse wird eine Färbung mittels Antikörper gegen das eingesetzte Virus angeschlossen. Dabei erfolgt eine prozentuale Abschätzung des CPE im Vergleich zur Viruskontrolle, die als 100% gesetzt wird. Bei der Färbung wird eine photometrische Auswertung durchgeführt. Mittels linearer Regression unter Verwendung eines Computerprogramms wird die Konzentration, bei der eine 50%ige Reduktion der Virusreplikation von Patientenisolaten bewirkt wird ("IC₅₀"), errechnet.

### Die Konzentration an Kondensationsprodukt, die die Zellviabilität um 50% reduziert, wird "TC₅₀" (= toxic concentration) genannt. TC₅₀ zeigt die toxischen Effekte des Reaktionsgemisches an.

Das Verhältnis TC₅₀ / IC₅₀ (therapeutisches Index "TI" genannt) zeigt die spezifische Aktivität eines Kondensationsproduktes gegen ein gegebenes Virus.

### Endpunkttitration des Virus

### Vorbereitung und Verteilung der Zellsuspensionen

### 1. Allgemein

- aus einer Vero-Zellkulturflasche (75cm², ca. 9x10⁶ Zellen) mit konfluentem Zellrasen können ca. 100ml Zellsuspension hergestellt werden.
- pro Mikrotiterplatte werden 5ml Zellsuspension benötigt

### 2. Herstellung der Zellsuspensionen

- die Zellkultur wird trypsiniert, homogenisiert und in Wachstumsmedium überführt

### 3. Verteilung der Zellsuspension

- 50µl Zellsuspension pro Loch ansetzen
- Platten dann bis zur weiteren Benutzung im Brutschrank aufbewahren

### Titration der Viren

- Herstellung einer 1:10 Verdünnungsreihe
- jeweils 50µl Verdünnung pro Loch im 8-fach Ansatz in eine mit Zellsuspension vorbereitete Platte pipettieren
- je nach Virus die Platte im Brutschrank für einige Tage inkubieren
- nach der Inkubationszeit Platten mikroskopisch nach CPE beurteilen

### Testvorbereitung zur Bestimmung der antiviralen Aktivität

- Pro zu untersuchendes Virus muss eine 96-Loch Platte mit entsprechenden Zellen vorbereitet werden
- Beschriftung der Platten.
   ➢ 1. Reihe ZK (Zellkontrolle)
   ➢ 2. Reihe VK (Viruskontrolle)
   ➢ 3. - 10. Reihe Stoffkonzentrationen
   ➢ am Rand Virus und Datum

### Testdurchführung

mit der zu testenden Substanz eine 1:2 Verdünnungsreihe herstellen
- In Reihe 1 der Platte 100µl Medium zu den Zellen geben (keine Stoff- und Virusverdünnung)
- In Reihe 2 50µl Medium pipettieren (keine Stoffverdünnung)
- Substanzverdünnung Reihe 3-12 im 8-fach Ansatz auf die Platte verteilen

*Das Einpipettieren in die Platten zügig durchführen, da sonst die Zellen austrocknen*
- eine ausreichende Menge Virussuspension für i.d.R. MOI 0,01 herstellen
- 50µl Virussuspension ab Reihe 2 pipettieren
- Platte bei 37°C im CO₂-Bruschrank für 2 Tage inkubieren

### Testauswertung

- Nach Ende der Inkubationszeit die Platte mikroskopisch auf CPE auswerten. Dabei entspricht die Viruskontrolle 100%. Für alle Substanzverdünnungen wird, durch Vergleich mit der Viruskontrolle, der Umfang der Zellzerstörung als Prozentzahl angegeben. Nach der visuellen Auswertung kann eine Färbung angeschlossen werden

### Färbung mit virusspezifischen Antikörpern

unter der Sterilwerkbank Überstand aus Mikrotiterplatten absaugen
- Fixation der Zellen mit Aceton/Methanol
- Flüssigkeit absaugen
- Verdünnung der virusspezifischen Antikörper mit Blocking Solution. Pro Kavität werden 50µl eingesetzt. Die optimale Konzentration für den Antikörper wird für jede neue Charge mittels Titration ermittelt. Inkubation 1h bei 37°C
- 3x Waschen mit Waschpuffer
- biotinylierte Anti-IgG-Antikörper werden in Waschpuffer verdünnt und je 50µl in jede Kavität einpipettiert. Die optimale Konzentration für den Antikörper wird für jede neue Charge mittels Titration ermittelt.
- Inkubation 1h 37°C
- 3x Waschen
- das Streptavidin/Peroxidase Konjugat wird im Waschpuffer verdünnt und 50µl pro Kavität eingesetzt. Die optimale Konzentration des Konjugates wird für jede neue Charge mittels Titration ermittelt.
- Inkubation für 30Min bei 37°C
- 3x Waschen
- in jede Kavität werden 50µl Substrat-Lösung einpipettiert
- bei Gebrauch eines löslichen Substrates auf einer separaten Platte 2 Reihen mit 50µl Substrat pipettieren (=Substratleerwert)
- Inkubation 10Min bei RT
- Zum Abstoppen bei löslichem Substrat 100ml 1 N Schwefelsäure zugeben
- Auswertung photometrisch bei einer Wellenlänge von 450nm und einer Referenzwellenlänge von 630nm
- die Auswertung sollte innerhalb einer Stunde nach Ende des Tests erfolgen

### Kriterien zur analytischen Freigabe der Ergebnisse:

- die Zellkontrolle muss einen konfluenten, morphologisch unauffälligen Zellrasen zeigen
- die Viruskontrolle wird als 100% CPE gesetzt
- bei der Berechnung des IC₅₀-Wertes darf "r" nicht kleiner als 0,90 sein

### Auswertung und Dokumentation

### visuelle Auswertung

- bei der visuellen Auswertung wird die Ausprägung des CPE bzw. der Färbung im Vergleich mit der Viruskontrolle abgelesen und als %-Wert protokolliert
- Dabei muss die ZK einen konfluenten Zellrasen aufweisen
- grundsätzlich alle Vertiefungen begutachten

### photometrische Auswertung

- bei der photometrischen Auswertung wird aus allen 8-fach Bestimmungen jeweils ein Mittelwert errechnet
- der Mittelwert der Substratleerwerte wird von allen errechneten Mittelwerten subtrahiert
- der OD-Wert der VK entspricht 100%
- mittels Dreisatzrechnung werden die %-Werte der eingesetzten Stoffverdünnungen berechnet

### Berechnung des IC₅₀-Wertes

- aus den 8 Einzelwerten der Kontrollen bzw. der Substanzbestimmungen den jeweiligen Mittelwert ermitteln
- den Substratleerwert von allen Werten subtrahieren
- bei der antiviralen Bestimmung entspricht der Wert der VK 100%
- für die einzelnen Werte der Substanzbestimmungen den %-Wert in Bezug auf die jeweilige Kontrolle berechnen
- ermittelte %-Werte in das Computerprogramm Calcusyn for Windows (Fa. Biosoft) einsetzen und den IC₅₀-Wert berechnen.

### Qualitätskontrolle

In jedem Ansatz wird eine Substanz mit bekannter antiviraler Aktivität gegenüber dem Prüfvirus mitgeführt. In jedem Ansatz wird eine Zell- und Viruskontrolle mitgeführt.

### Beispiele antiviraler Aktivität von Kondensationsprodukten

### Kondensationsprodukt aus Beispiel1

| **Virus** | **IC₅₀ (µg/ml)** | **TC₅₀ (µg/ml)** | **TI** |
|---|---|---|---|
| Herpes simplex Virus 1 (HSV-1) | 1,7 | 1670 | 982 |
| Herpes simplex Virus 2 (HSV-2) | 0,9 | 1670 | 1856 |
| HCMV | 0,3 | 1670 | 5567 |
| Humanes Immundefizienzvirus Typ 1 (HIV-1) | 0,7 | 305 | 436 |

### Kondensationsprodukt aus Beispiel 2

| **Virus** | **IC₅₀ (µg/ml)** | **TC₅₀ (µg/ml)** | **TI** |
|---|---|---|---|
| Herpes simplex Virus 1 (HSV-1) | 0,89 | 872 | 980 |
| Herpes simplex Virus 2 (HSV-2) | 0,77 | 872 | 1132 |
| HCMV | 0,33 | 872 | 2642 |

### Kondensationsprodukt aus Vergleichsbeispiel 3

| **Virus** | **IC₅₀ (µg/ml)** | **TC₅₀ (µg/ml)** | **TI** |
|---|---|---|---|
| Herpes simplex Virus 1 (HSV-1) | 2500 | 500 | 0,2 |
| Herpes simplex Virus 2 (HSV-2) | 1500 | 500 | 0,3 |

### Kondensationsprodukt aus Vergleichsbeispiel 4

| **Virus** | **IC₅₀ (µg/ml)** | **TC₅₀ (µg/ml)** | **TI** |
|---|---|---|---|
| Herpes simplex Virus 1 (HSV-1) | 1,34 | 885 | 660 |
| Herpes simplex Virus 2 (HSV-2) | 0,87 | 885 | 1017 |
| HCMV | 0,42 | 885 | 2107 |

### Kondensationsprodukt aus Vergleichsbeispiel 5

| **Virus** | **IC₅₀ (µg/ml)** | **TC₅₀ (µg/ml)** | **TI** |
|---|---|---|---|
| Herpes simplex Virus 1 (HSV-1) | 2,85 | 632 | 220 |
| Herpes simplex Virus 2 (HSV-2) | 2,40 | 632 | 263 |
| HCMV | 1,88 | 632 | 336 |

### Kondensationsprodukt aus Vergleichsbeispiel 6

| **Virus** | **IC₅₀ (µg/ml)** | **TC₅₀ (µg/ml)** | **TI** |
|---|---|---|---|
| Herpes simplex Virus 1 (HSV-1) | 10,4 | 99,2 | 9,5 |
| HCMV | 1,59 | 99,2 | 62,39 |
| Humanes Immundefizienzvirus Typ 1 (HIV-1) | 10,8 | 405 | 37,7 |

Der Vergleich von Beispiel 1 und Vergleichsbeispiel 6 zeigt, dass die erfindungsgemäßen Kondensationsprodukte für den HSV-1Virus einen verringerten IC₅₀-Wert und einen deutlich höheren TC₅₀-Wert und somit auch einen deutlich verbesserten TI-Indes aufweisen als die aus dem Stand der Technik bekannten Kondensationsprodukte. Das erfindungsgemäße Kondensationsprodukt 3 zeigt für den HSV-1-Virus einen deutlich höheren TC₅₀-Wert als Vergleichsbeispiel 6, in dem keine Komponente a1) als Edukt (Reaktand) des Kondensationsproduktes enthalten ist. Die erfindungsgemäßen Kondensationsprodukte zeigen somit eine verbesserte pharmazeutische Wirksamkeit.

### Hemmung des Enzyms Humane Leukozyten Elastase (HLE)

Zum Nachweis einer Veränderung der Enzym-Aktivität wird HLE zusammen mit dem synthetischen Substrat N-Methoxysuccinyl-Ala-Ala-Pro-Val-pNitro-Anilid (AAPV) inkubiert. Der Umsatz von AAPV wird dabei photometrisch bei 405 nm bestimmt. Zu dem Ansatz werden die Kondensationsprodukte aus den Beispielen in unterschiedlichen Konzentrationen gegeben. Es zeigt sich eine Dosis-abhängige Hemmung der HLE-Aktivität der Kondensationsprodukte von Beispiel 1 und Vergleichsbeispiel 6. Dabei beträgt die halb-maximale effektive Konzentration (EC₅₀) für Beispiel 1 0,3 µg/ml, für Vergleichsbeispiel 6. beträgt die EC₅₀ 0,4 µg/ml.

### Wirkung der Kondensationsprodukte bei einem Tiermodell der chronisch obstruktiven Lungenerkrankung (COPD)

Zur Untersuchung der Wirkung Beispiel 1 in einem Modell zur chronisch obstruktiven Lungenerkrankung (COPD) werden männliche syrische Goldhamster (mittleres Gewicht 125g ± 6 g) in zwei Gruppen (Verumgruppe und Kontrollgruppe) zu je 10 Hamstern aufgeteilt. Die Tiere beider Gruppen werden mittels intraperitoneal verabreichtem Ketamin (95 mg/kg) und Xylazin (17 mg/kg) anästhesiert und anschließend endotracheal intubiert. Die Verumgruppe erhält 250 µl einer Lösung (0,5 µg/ml) von Beispiel 1 endotracheal in das Bronchialsystem eingebracht, der Kontrollgruppe wird 250 µl isotonische Kochsalzlösung verabreicht. Nach 30 Minuten erfolgt für beide Gruppen eine zweite Instillation mit 100 µl humaner Leukozyten Elastase (HLE, 500 µg/ml in steriler 0.9% NaCl-Lösung). Nach 4 Stunden (6 Tiere pro Gruppe) bzw. nach 24 Stunden (4 Tiere pro Gruppe) nach der letzten Instillation werden die Tiere getötet (Phenobarbital 70 mg/kg intraperitoneal). Für die 4 Stunden nach der Instillation getöteten Tiere wird eine BAL (broncho-alveoläre Lavage, BAL) durchgeführt. Für die BAL wird 2,5 ml 0,9% NaCl-Lösung wiederholt (insgesamt 3mal) verwendet und anschließend die Hämoglobinkonzentration im Instillat spektrophotometrisch bestimmt. Dieser Wert wird als Maß für die Schädigung der Lunge durch Blutaustritt (Hämorrhagie) durch HLE verwendet.

### Wirkung von Aerosolen der Kondensationsprodukte bei einem Tiermodell der COPD (I)

Männliche syrische Goldhamster (mittleres Gewicht 105g ± 8 g) werden in zwei Gruppen (Verumgruppe und Kontrollgruppe) zu je 6 Hamstern aufgeteilt. Die Tiere beider Gruppen werden zunächst intraperitoneal anästhesiert (Ketamin 95 mg/kg, and Xylazin 17 mg/kg) und anschließend mittels direkter Laryngoskopie endotracheal intubiert. Beide Gruppen erhalten 100 µl HLE (500 µg/ml in steriler 0.9% NaCl-Lösung) instilliert. Anschließend werden die Tiere in getrennten Käfigen mit einer kontinuierlichen Behandlung mit einem Aerosol von Beispiel 1 (5 µg/ml) (Verumgruppe) oder isotoner Kochsalzlösung (Kontrollgruppe) in der Atemluft (Luftfeuchtigkeit 60%) für 24 Stunden behandelt. Nach dieser Behandlung werden die Tiere getötet (Phenobarbital 70 mg/kg intraperitoneal) und eine BAL (bronchoalveoläre Lavage) durchgeführt. Für die BAL wird 2,5 ml 0,9% NaCl-Lösung wiederholt (insgesamt 3mal) instilliert und anschließend die Hämoglobinkonzentration im Instillat spektrophotometrisch bestimmt. Der so erhaltene Wert wird als Maß für die Schädigung der Lunge (Hämorrhagie) durch die Serinprotease (HNE) benutzt.

### Wirkung von Aerosolen der Kondensationsprodukte bei einem Tiermodell der COPD (II)

Männliche syrische Goldhamster (mittleres Gewicht 115g ± 10 g) werden für diese Versuche verwendet. Die Tiere werden zunächst intraperitoneal anästhesiert (Ketamin 95 mg/kg, Xylazin 17 mg/kg) und anschließend endotracheal intubiert. Alle Tiere erhalten 100 µl humaner Leukozyten-Elastase (500 µg/ml in steriler 0.9% NaCl-Lösung) endotracheal instilliert. Anschließend werden jeweils 4 Tiere in getrennten Käfige (je 2 Hamster pro Käfig) untergebracht und einer dauernden Behandlung mit einem Aerosol von Beispiel 1 (Konzentration der Kondensationsprodukte von Beispiel 1 ist 5 µg/ml) oder Wasser-Aerosol in der Atemluft (Luftfeuchtigkeit 60%) für 4 Stunden unterzogen. Nach dieser Behandlung werden alle Tiere getötet (Phenobarbital 70 mg/kg intraperitoneal) und eine BAL (bronchoalveoläre Lavage) durchgeführt. Für die BAL wird 2,5 ml 0,9% NaCl-Lösung wiederholt (insgesamt 3mal) instilliert und anschließend die Hämoglobinkonzentration im Instillat spektrophotometrisch bestimmt. Dieser Wert wird als Maß für die Schädigung der Lunge (Hämorrhagie) durch die HLE gewertet.

### Wirkung der Kondensationsprodukte in einem Modell zum Reperfusionsschaden des Myokards

Der Reperfusionsschaden wird bei männlichen, weißen Neuseeland-Kaninchen durch Ligatur einer Koronararterie eine Minderdurchblutung des Herzmuskelgewebes erreicht (Bidouard JP et al., Eur J Pharmacol. 2003;461:49-52). Dabei wird durch Thorakotomie ein Seitenast der linken Koronararterie mittels Ligatur für 30 Minuten verschlossen und anschließend wieder eröffnet um eine Reperfusion über 120 Minuten zu erreichen. Vor Entnahme des Herzens wird Farbtusche in die Aorta ascendenz gegeben. Zur Untersuchung der Wirkung von Beispiel 1 auf den Reperfusionsschaden werden die Tiere in 3 Gruppen zu je 3 Tieren eingeteilt. Je eine Gruppe erhält 15 Minuten vor (Gruppe 1) oder 25 Minuten nach (Gruppe 2) der Wiedereröffnung der Koronararterie eine intravenöse Behandlung mit Beispiel 1 (5 µg/ml in isotoner Kochsalzlösung, eine dritte Gruppe (Kontrollgruppe) wird mit isotoner Kochsalzlösung alleine behandelt.

Nach Tötung der Tiere werden die Herzen entnommen, mit Formalin fixiert und in Paraffin eingebettet. Anhand entsprechender Schnitte wird das Herz auf die Verteilung der Tusche untersucht. Ferner werden neutrophile Granulozyten, die das Enzym HLE enthalten, mittels alpha-Naphthol-Chloracetat-Esterase-Färbung im histologischen Schnitt dargestellt.

### Hemmung des Ischämieschadens im Hirn durch die Kondensationsprodukte

Die Wirkung von Beispiel 1 auf den cerebralen Ischämieschaden wird in einem Tiermodell untersucht. Erwachsenen Long-Evans Ratten werden durch Verschluss beider A. carotis für eine Stunde eine cerebrale Ischämie zugefügt, der nach Wiedereröffnung der Gefäße eine Reperfusion über 24 Stunden folgt. Die Evaluierung des Schädigungsgrades erfolgt anhand neuro-vegetativer Symptome (Spontanaktivität, koordiniertes Gehen, Vorfußstrecken und Klettern). Die Schädigung des Hirngewebes wird ebenfalls histologisch untersucht. In zwei Versuchsgruppen zu je 3 Ratten werden den Tieren 15 Minuten vor Einleitung der Ischämie und unmittelbar vor der folgenden Reperfusion entweder Beispiel 1 (5 µg/ml)- (Verumgruppe) oder isotone Kochsalzlösung-(Kontrollgruppe) infundiert.

### Wirkung der Kondensationsprodukte bei bullösem Pemphigoid

Bei Patienten mit bullösem Pemphigoid wird die Blasenflüssigkeit entnommen und für ein ex vivo Modell verwendet (Verraes et al., J Invest Dermatol. 2001;117:1091-6). Anschließend wird rekombinantes, radioaktiv markiertes BP180-Antigen, das Zielantigen bei dieser Erkrankung, der Blasenflüssigkeit zugesetzt und der Abbau autoradiographisch nach Gelelektrophorese bestimmt. Zur Hemmung der Elastase-Aktivität in der Blasenflüssigkeit wird entweder Beispiel 1 (5 µg/ml) oder der Elastase-Inhibitor Chloromethylketon zugesetzt. Zum Vergleich dient eine unbehandelte Kontrolle.

### Wirkung der Kondensationsprodukte bei Psoriasis vulgaris

Die Psoriasis ist durch Nachweis von neutrophilen Granulozyten in die obersten Hautschichten charakterisiert. Hohe Aktivität von HLE lässt sich auf der läsionären Haut nachweisen (Wiedow et al., J Invest Dermatol. 1992;99:306-9).

Zur Bestimmung der Wirkung von Beispiel 1 bei Psoriasis vulgaris wird ein Halbseitenversuch durchgeführt. Hierzu wird Beispiel 1 in einer Konzentration von 50µg/mg in Basiscreme DAC verwendet. Es werden Patienten eingeschlossen, die vergleichbare Läsionen an beiden Ellenbogen aufwiesen. Zur Evaluierung wird der "local psoriasis severity index" (LPSI) verwendet (Henneicke-von Zepelin et al., Br J Dermatol. 1993;129:713-7). Die Patienten werden angewiesen, den Inhalt der mit "rechts" und "links" markierten Tuben 2x/Tag auf die entsprechenden Läsionen aufzutragen. In der einen Tube befindet sich die Beispiel 1-haltige Creme, in der anderen Basiscreme DAC ohne Zusatz. Die Zuteilung erfolgt zufällig. Der LPSI wird einmal alle 2 Wochen bestimmt.

### Wirkung der Kondensationsprodukte bei Peritonitis und septischen Schock

In einem Tiermodell zur infektiösen Peritonitis wird die Wirkung von Beispiel 1 zur Anwendung bei einer Peritonealdialyse untersucht (Welten et al., Nephrol Dial Transplant. 2004;19:831-9.). Hierfür wird männlichen Wistar-Ratten ein Katheder zur Peritonealdialyse gelegt und zur Auslösung einer Peritonitis sowie zur weiteren Peritonealdialyse verwendet. Eine eitrige Peritonitis wird durch Einspülung einer Suspension von S. aureus ATCC 25923 (1 x 109 c.f.u in 0.5 ml) erzeugt. Nach 4 Stunden wird eine peritoneale Lavage entweder mit isotoner Kochsalzlösung oder mit einer isotonen Kochsalzlösung mit 5 µg/ml Beispiel 1 durchgeführt. Nach dem Tod der Tiere spätestens aber nach 24 Stunden wird die bakterielle Besiedlung sowie die entzündliche Reaktion im Peritoneum histologisch untersucht.

### Wirkung der Kondensationsprodukte auf die allergene Potenz von Typ-1-Allergenen

Ein Extrakt der Hausstaubmilbe Dermatophagoides pteronissinus (D. pter) wird mit Beispiel 1 (Endkonzentration 50 µg/ml) behandelt. Anschließend wird der D. pter/Beispiel 1-Extrakt in einem Pric-Test bei Patienten untersucht, die auf die konventionelle Testflüssigkeit mit D. pter positiv reagiert haben. Zur Kontrolle dient erneut die unbehandelte Testlösung, Histamin-Lösung 0.1%, mit Beispiel 1-behandelte Histamin-Lösung sowie isotone Kochsalzlösung.

### Wirkung eines mit Kondensationsprodukten beschichtetem Baumwolltuch auf nächtliche Symptome von Patienten mit nachgewiesener Typ-1-Sensibilisierung gegenüber Hausstaubmilben (D. pteronissinus)

Matratzen stellen eine wesentliche Quelle der von Hausstaubmilben verursachten Allergien vom Soforttyp (Typ-1-Allergie) dar. Bei den betroffenen Patienten äußert sich dies in nächtlichen Niesanfällen, Augentränen und Fließschnupfen. Zur Prophylaxe werden häufig Allergen-dichte Matratzenbezüge verwendet.

In einem Versuch wird dichtes Baumwollgewebe mit einer Beispiel 1-haltigen Beschichtung versehen. Jeweils 10 Patienten mit nachgewiesener Typ-1-Sensibilisierung gegenüber D. Pter (pric-Test; Nachweis von spezifischem IgE, mindestens CAP-Klasse 3) werden in die Untersuchung einbezogen. Sie erhalten randomisiert mit Beispiel 1-beschichtete oder unbehandelte Matratzenbezüge. Der Effekt wird mittels eines standardisierten Patiententagebuches festgehalten. Dabei werden Häufigkeit nächtlichen Aufwachens, Zahl der Niesanfälle und mittels visueller Analogskalen Intensität von Fließschnupfen und Augentränen erfasst.

### Wirkung von Kondensationsprodukt-haltigem Nasenspray bei Patienten mit allergischer Rhinitis

In einem Versuch wird bei Patienten mit nachgewiesener allergischer Rhinitis gegen Gräserpollen entweder ein Nasenspray verwendet, das 50 µg/ml Beispiel 1 enthält, oder isotonische Kochsalzlösung. Jeweils 5 Patienten pro Gruppe werden angewiesen, das Spray 6x/Tag zu verwenden.

### Wirkung von Kondensationsprodukt haltigen Augentropfen bei Patienten mit allergischer Konjunktivitis

In einem Versuch werden bei Patienten mit nachgewiesener allergischer Konjunktivitis entweder Augentropfen verwendet, dass 5 µg/ml Beispiel 1 enthält, oder isotonische Kochsalzlösung. Jeweils 5 Patienten pro Gruppe werden angewiesen, die Tropfen 6x/Tag zu verwenden.

### Wirkung von Kondensationsprodukt-haltigem Aerosol bei allergischem Asthma bronchiale

In einem Tiermodell bei 10-Wochen alten braunen Norwegischen Ratten wird die Wirkung von Beispiel 1 überprüft (Roumestan et al., Resp Res 8:35-46, 2007). Durch wiederholte intraperitoneale Injektionen von Ovalbumin wird eine Sensibilisierung der Tiere erreicht. Die Tiere werden dann in einen klimatisierten Käfig überführt und konstant mit einem Aerosol aus isotoner Kochsalzlösung oder Beispiel 1-Lösung für 6 Tage behandelt. Daran anschließend erfolgt eine Asthma-Auslösung durch ein Ovalbuminhaltiges Aerosol. Für die Messung der Wirksamkeit wird die Veränderung des Atemwegswiderstandes durch barometrische Plethysmographie an den lebenden Tieren nach 24 h bestimmt.

### Anwendung von Kondensationsprodukt-Infusionen bei systemischen Vaskulitiden am Beispiel der Wegenerschen Granulomatose.

Bei der Wegenerschen Granulomatose kommt es zu einer Aktivierung von neutrophilen Granulozyten die damit eine wichtige Bedeutung für die Pathogenese haben. Die Krankheitsaktivtät kann durch Bestimmung von. Proteinase 3-anti-neutrophilen cytoplasmatischen Antikörpern (PR3-ANCA) bestimmt werden.

In einer offenen Studie wird bei Patienten mit M. Wegener die Wirkung einer Infusion mit Beispiel 1 einmal täglich über eine Woche zusätzlich zur individuellen systemischen Therapie bei 5 Patienten untersucht. Die Bestimmung des PR3-ANCA im Serum vor und nach einer Woche nach der letzten Beispiel 1-haltigen Infusion dient als Kontrollparameter. Fünf Patienten, bei denen ausschließlich die individuelle Therapie ohne weitere Infusionen angewendet wird, dienen als Vergleich.

## Patentansprüche

1. Kondensationsprodukt erhältlich durch Umsetzung von
a1) 1-Naphthalincarbonsäure oder einem Salz davon,
a2) mindestens einem Aldehyd ausgewählt aus Formaldehyd, Acetaldehyd und Propionaldehyd
a3) konzentrierter Schwefelsäure
a4) mindestens einem Harnstoffderivat ausgewählt aus Harnstoff, Melamin, 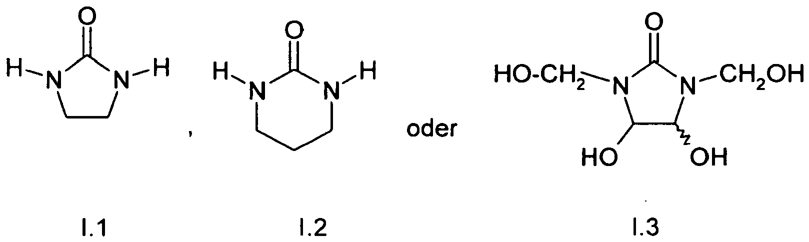 und
a5) gegebenenfalls Phenol
oder ein physiologisch verträgliches Salz davon.

2. Kondensationsprodukt gemäß Anspruch 1, **dadurch gekennzeichnet, dass** die Komponente a5) vorhanden ist.

3. Kondensationsprodukt gemäß Anspruch 1 oder 2, **dadurch gekennzeichnet, dass** das Kondensationsprodukt als Gemisch mit mindestens einem Gerbstoff, insbesondere einem synthetischen oder pflanzlichen Gerbstoff vorliegt.

4. Kondensationsprodukt gemäß einem der Ansprüche 1 bis 3 zur Verwendung als Arzneimittel.

5. Verwendung eines Kondensationsproduktes nach einem der Ansprüche 1 bis 3 zur Herstellung von einem Medikament zur Prophylaxe und/oder Behandlung von Genitalwarzen, Gebärmutterhalskrebs, allergischen oder nicht-allergischen Ekzemen, insbesondere Neurodermitis (endogenes Ekzem), Wundsein, Juckreiz, bullösem Pemphigoid, Psoriasis vulgaris; der allergenen Potenz von Typ 1-Allergenen, allergischer Rhinitis, allergischer Konjunktivitis, allergischem Asthma Bronchiale, Schuppenflechte, entzündlichen Erkrankungen, Autoimmunerkrankungen, insbesondere Arthritis, Rheuma, melanomen Karzinomen, Entzündungen der Haut, Herpes, insbesondere Herpes labilis oder Herpes simplex, Windpocken, Aids, Lungenerkrankungen, Myokard-Reperfusion, Ischämieschaden des Hirns, Peritonitis, septischem Schock oder systemischen Vaskulitiden.

6. Verwendung eines Kondensationsproduktes nach einem der Ansprüche 1 bis 3 zur Herstellung von einem Medikament, das ein antivirales Agens ist, vorzugsweise gegenüber humanen Papillomviren, speziell Typ 16, 18, 6 und 11, endogenen Retroviren, insbesondere HERV-Typ, Herpes-Viren, HCMV-Viren, HIV-Viren, Corona-Viren, Flaviviren, Togaviren oder Paramoxyviren.

7. Verwendung eines Kondensationsproduktes nach einem der Ansprüche 1 bis 3 zur Inhibierung von Serin Proteasen, insbesondere der Humanen Leukozyten Elastase (HLE) oder der Proteinase 3.

8. Pharmazeutische Zusammensetzung umfassend eine wirksame Menge von mindestens einem Kondensationsprodukt gemäß einem der Ansprüche 1 bis 3 und einem physiologisch verträglichen Träger.

9. Pharmazeutische Zusammensetzung gemäß Anspruch 8, wobei die pharmazeutische Zusammensetzung in Form einer Pille, Tablette, Lutschtablette, Granulat, Kapsel, harte oder weiche Gelatinekapsel, wässrigen Lösung, alkoholischen Lösung, ölartigen Lösung, Sirup, Emulsion, Suspension, Zäpfchen, Pastille, Lösung zur Injektion oder Infusion, Salbe, Tinktur, Creme, Lotion, Puder, Spray, eines transdermalen therapeutischen Systems, Nasenspray, Aerosol, Aerosol-Mischung, Mikrokapsel, Implantat, Stab, Pflaster oder Gel vorliegt oder dass die pharmazeutische Zusammensetzung Bestandteil von Health Care-Produkten wie Sonnenschutzcremes, Nasensprays, Mundwässer, Zahnpasten, Pflastern, (Feucht)-Tüchern, Waschlotionen oder Shampoos ist.

10. Verwendung eines Kondensationsproduktes gemäß einem der Ansprüche 1 bis 3 zur Desinfektion, als Desinfektionsmittel oder Bestandteil eines Desinfektionsmittels.

11. Verwendung gemäß Anspruch 10 im Bereich von Krankenhäusern, insbesondere Krankenhaus-Intensivstationen, Toiletten, Waschräumen, Haushalten, der Lebensmittelproduktion oder in Stallungen oder Käfigen von Tieren, insbesondere von Vögeln, Schweinen oder Rindern.

12. Desinfektionsmittel enthaltend mindestens ein Kondensationsprodukt gemäß einem der Ansprüche 1 bis 3.

13. Verfahren zur Herstellung eines Kondensationsproduktes gemäß einem der Ansprüche 1 bis 3, **dadurch gekennzeichnet, dass** die einzelnen Komponenten a1), a2), a3) und a4) sowie gegebenenfalls a5) in einer oder in mehreren Stufen miteinander umgesetzt werden.

14. Verwendung eines Kondensationsproduktes gemäß einem der Ansprüche 1 bis 3 als Gerbstoff.

## Claims

1. A condensation product obtainable by reaction of
a1) 1-naphthalenecarboxylic acid or a salt thereof,
a2) at least one aldehyde selected from formaldehyde, acetaldehyde and propionaldehyde
a3) concentrated sulfuric acid
a4) at least one urea derivative selected from urea, melamine, 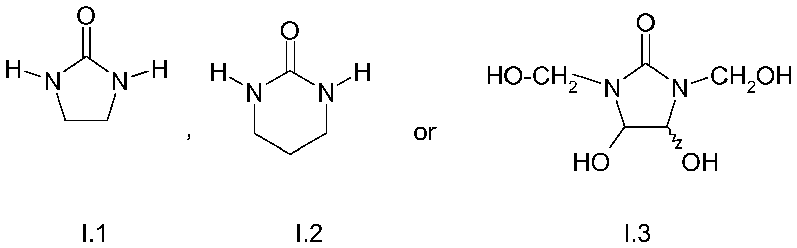 and
a5) optionally phenol
or a physiologically tolerable salt thereof.

2. The condensation product according to claim 1, wherein component a5) is present.

3. The condensation product according to claim 1 or 2, wherein the condensation product is present as a mixture with at least one tanning agent, in particular a synthetic or plant tanning agent.

4. The condensation product according to one of claims 1 to 3 for use as a drug.

5. The use of a condensation product according to one of claims 1 to 3 for the production of a medicament for the prophylaxis and/or treatment of genital warts, cancer of the uterine cervix, allergic or nonallergic eczema, in particular neurodermatitis (endogenous eczema), diaper rash, pruritus, bullous pemphigoid, psoriasis vulgaris, the allergenic potency of type 1 allergens, allergic rhinitis, allergic conjunctivitis, allergic bronchial asthma, psoriasis, inflammatory diseases, autoimmune diseases, in particular arthritis, rheumatism, melanomatous carcinomas, inflammations of the skin, herpes, in particular herpes labilis or herpes simplex, chickenpox, Aids, lung diseases, myocardial reperfusion, ischemic brain damage, peritonitis, septic shock or systemic vasculitides.

6. The use of a condensation product according to one of claims 1 to 3 for the production of a medicament which is an antiviral agent, preferably against human papillomaviruses, especially type 16, 18, 6 and 11, endogenous retroviruses, in particular HERV type, herpes viruses, HCMV viruses, HIV viruses, coronaviruses, flaviviruses, togaviruses or paramyxoviruses.

7. The use of a condensation product according to one of claims 1 to 3 for inhibiting serine proteases, in particular human leucocyte elastase (HLE) or proteinase 3.

8. A pharmaceutical composition comprising an efficacious amount of at least one condensation product according to one of claims 1 to 3 and a physiologically tolerable vehicle.

9. The pharmaceutical composition according to claim 8, where the pharmaceutical composition is present in the form of a pill, tablet, lozenge, granules, capsule, hard or soft gelatin capsule, aqueous solution, alcoholic solution, oily solution, syrup, emulsion, suspension, suppository, pastille, solution for injection or infusion, ointment, tincture, cream, lotion, cosmetic powder, spray, of a transdermal therapeutic system, nasal spray, aerosol, aerosol mixture, microcapsule, implant, rod, patch or gel or wherein the pharmaceutical composition is a constituent of healthcare products such as sunscreen creams, nasal sprays, mouthwashes, toothpastes, plasters, (wet) wipes, cleansing lotions or shampoos.

10. The use of a condensation product according to one of claims 1 to 3 for disinfection, as a disinfectant or constituent of a disinfectant.

11. The use according to claim 10 in the hospital field, in particular hospital intensive care units, toilets, washrooms, households, food production or in stables or cages of animals, in particular of birds, pigs or cattle.

12. A disinfectant comprising at least one condensation product according to one of claims 1 to 3.

13. A process for the preparation of a condensation product according to one of claims 1 to 3, wherein the individual components a1), a2), a3) and a4) and optionally a5) are reacted with one another in one or in a number of stages.

14. The use of a condensation product according to one of claims 1 to 3 as a tanning agent.

## Revendications

1. Produit de condensation pouvant être obtenu par mise en réaction
a1) d'acide 1-naphtalènecarboxylique ou d'un sel de celui-ci,
a2) d'au moins un aldéhyde choisi parmi le formaldéhyde, l'acétaldéhyde et le propionaldéhyde
a3) d'acide sulfurique concentré
a4) d'au moins un dérivé d'urée choisi parmi l'urée, la mélamine, 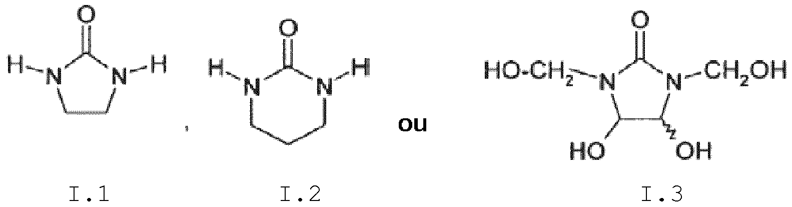 et
a5) éventuellement de phénol
ou un sel physiologiquement acceptable de celui-ci.

2. Produit de condensation selon la revendication 1, **caractérisé en ce que** le composant a5) est présent.

3. Produit de condensation selon la revendication 1 ou 2, **caractérisé en ce que** le produit de condensation se trouve sous forme de mélange avec au moins un tanin, en particulier un tanin végétal ou synthétique.

4. Produit de condensation selon l'une quelconque des revendications 1 à 3, pour utilisation en tant que médicament.

5. Utilisation d'un produit de condensation selon l'une quelconque des revendications 1 à 3, pour la fabrication d'un médicament destiné à la prophylaxie et/ou au traitement de verrues génitales, cancer du col utérin, eczémas allergiques ou non allergiques, notamment névrodermite (eczéma endogène), intertrigo, prurit, pemphigoïde bulleux, psoriasis vulgaire, allergènes de pouvoir allergénique du type 1, rhinite allergique, conjonctivite allergique, asthme bronchique allergique, psoriasis, maladies inflammatoires, maladies auto-immunes, en particulier arthrite, rhumatisme, carcinomes mélanomiques, inflammations de la peau, herpès, notamment herpès de la lèvre ou herpes simplex, varicelle, sida, maladies pulmonaires, reperfusion du myocarde, lésions ischémiques du cerveau, péritonite, choc septique ou vascularites systémiques.

6. Utilisation d'un produit de condensation selon l'une quelconque des revendications 1 à 3, pour la fabrication d'un médicament qui est un agent antiviral, de préférence contre des papillomavirus humains, en particulier les types 16, 18, 6 et 11, des rétrovirus endogènes, en particulier le type HERV, des herpesvirus, virus HCMV, virus VIH, coronavirus, flavivirus, togavirus ou paramoxyvirus.

7. Utilisation d'un produit de condensation selon l'une quelconque des revendications 1 à 3, pour l'inhibition de sérine protéases, en particulier de l'élastase leucocytaire humaine (HLE) ou de la protéinase 3.

8. Composition pharmaceutique comprenant une quantité efficace d'au moins un produit de condensation selon l'une quelconque des revendications 1 à 3 et un véhicule physiologiquement acceptable.

9. Composition pharmaceutique selon la revendication 8, la composition pharmaceutique étant sous forme d'une pilule, d'un comprimé, d'une tablette à sucer, d'un granulé, d'une capsule, d'une gélule ou d'une capsule de gélatine molle, d'une solution aqueuse, d'une solution alcoolique, d'une solution huileuse, d'un sirop, d'une émulsion, d'une suspension, d'un suppositoire, d'une pastille, d'une solution pour injection ou perfusion, d'une pommade, d'une teinture, d'une crème, d'une lotion, d'une poudre, d'une composition à pulvériser en aérosol, d'un système thérapeutique transdermique, d'une composition pour pulvérisation nasale, d'un aérosol, d'un mélange pour aérosol, d'une microcapsule, d'un implant, d'une tige, d'un pansement ou d'un gel ou la composition pharmaceutique faisant partie de produits de soin de santé tels que des crèmes de protection solaire, produits pour pulvérisation nasale, bains de bouche, pâtes dentifrice, pansements, lingettes (humides), lotions de nettoyage ou shampooings.

10. Utilisation d'un produit de condensation selon l'une quelconque des revendications 1 à 3, pour la désinfection, en tant que désinfectant ou composant d'un désinfectant.

11. Utilisation selon la revendication 10, dans le secteur des hôpitaux, en particulier des unités de soins intensifs, des toilettes, des salles d'eau, de la maison, de la fabrication des produits alimentaires ou dans des bâtiments ou cages hébergeant des animaux, en particulier des oiseaux, porcins ou bovins.

12. Désinfectant contenant au moins un produit de condensation selon l'une quelconque des revendications 1 à 3.

13. Procédé pour la préparation d'un produit de condensation selon l'une quelconque des revendications 1 à 3, **caractérisé en ce qu'**on fait réagir entre eux les composants individuels a1), a2), a3) et a4) ainsi qu'éventuellement a5) en une ou en plusieurs étapes.

14. Utilisation d'un produit de condensation selon l'une quelconque des revendications 1 à 3, en tant que tanin.
